Europäisches Patentamt

European Patent Office

Office européen des brevets

(11) Publication number: **0 612 845 A2**

# EUROPEAN PATENT APPLICATION

(21) Application number: **94101968.9**

(22) Date of filing: **09.02.94**

(51) Int. Cl.5: **C12N 15/12**, C12N 5/10,
C12P 21/02, C07K 13/00,
C07K 3/20, A61K 39/395

(30) Priority: **26.02.93 US 26140**

(43) Date of publication of application:
**31.08.94 Bulletin 94/35**

(84) Designated Contracting States:
**AT BE CH DE DK ES FR GB GR IE IT LI LU NL PT SE**

(71) Applicant: **AMERICAN CYANAMID COMPANY**
**One Cyanamid Plaza**
**Wayne New Jersey 07470 (US)**

(72) Inventor: **Eppler, Cecil Mark**
**162 Bateman Road**
**Langhorne, Pennsylvania 19047 (US)**
Inventor: **Shieh, Hong-Ming**
**35 Pine Glen Road**
**Langhorne, Pennsylvania 19047 (US)**
Inventor: **Zysk, John Ronald**
**RD 2, Box 183,**
**Route 519**
**Frenchtown, New Jersey 08825 (US)**
Inventor: **Corbett, Martin John**
**112 Union Street**
**Mt. Holly, New Jersey 08060 (US)**

(74) Representative: **Wächtershäuser, Günter, Prof. Dr.**
**Patentanwalt**
**Tal 29**
**D-80331 München (DE)**

(54) **Purified opioid receptor.**

(57) The present invention relates to a substantially pure m-subtype opioid receptor, and the nucleotide sequence encoding this receptor.

EP 0 612 845 A2

## FIELD OF THE INVENTION

The present invention relates to substantially pure opioid receptors, and the nucleotide sequence encoding the opioid receptor.

## BACKGROUND OF THE INVENTION

Opioids are a chemically diverse group of compounds which includes naturally occurring peptides and alkaloids as well as a large number of synthetic analogs. The physiological effects of opioid agonists include analgesia, drowsiness, changes in mood, respiratory depression, decreased gastrointestinal motility, nausea, vomiting and alterations in the endocrine and autonomic nervous systems (Jaffe and Martin, in The Pharmacological Basis of Therapeutics, Gilman, A.G. et al., eds.; MacMillan, New York, pages 491-531, 1985).

Opioid Subtypes and Their Receptors

The physiological actions of opioids are mediated through specific receptors that exist in the responsive tissues. In vitro characterization of these receptors by binding of radiolabelled opioid alkaloids to brain membranes was first described in 1973 by three independent groups of investigators was used to searchfor endogenous opioids in vertebrate brain. This search led to the discovery of met- and leu-enkephalin, two opioid pentapeptides, in 1975. In that same year, other opioid peptides (derived from the gene later designated as "POMC") were discovered in the pituitary. Selective binding of the different peptide and nonpeptide opioids to the receptors in membranes derived from different tissues and brain regions plus correlations with pharmacological selectivities of the opioid drugs established the existence of opioid receptor subclasses (history reviewed by A. Goldstein in Opioids: Past, Present and Future, Collier, H.O.J. et al., eds.; Taylor and Frances Ltd., London, 1984, pages 127-143).

The best characterized classes of opiate receptors are the mu (m), delta (w) and kappa (k) classes, based on clear differences in their ligand selectivities and pharmacological effects (Lord et al., Nature, 267:495-499, 1977). Sigma (s) (Jaffe and Martin, supra), and epsilon e (Schulz et al., J. Pharmacol. Exp. Ther. 216:604-606, 1981) are also thought to exist, based on differential pharmacology and ligand binding. There is also evidence for receptor subtypes within these major classes (Jaffe and Martin, supra).

Mechanisms of Action of Opioids

The best characterized effects of opioids on cell metabolism are decreased $Ca^{2+}$ conductance, increased $K^+$ conductance and decreased levels of cAMP (Loh and Smith, Ann. Rev. Pharmacol. Toxicol., 30:123-147, 1990). These functions are among those known to be regulated by the receptor-associated G proteins, which also confer high-affinity ligand binding on the receptors they associate with (Birnbaumer et al., Biochem. Biophys, Acta. 1031:163-224, 1990). In fact, both the binding of opiate agonists and their effects on adenylate cyclase have been shown to be GTP-dependent. It is likely that a thorough understanding of the signaling mechanisms of opiate receptors, including the identification of specific, receptor-associated G proteins, would shed light on underlying the mechanisms of functions such as analgesia and addiction.

Numerous previous attempts have been made to isolate opioid receptors. Bidlack et al. (PNAS USA 78:636-639, 1981) disclose the isolation of three species in the molecular weight range of 25-50,000 daltons, isolated from rat brain using affinity chromatography with 14-b-bromoacetamido-morphine. Gioannini et al. (J. Biol. Chem. 260:15117-15121, 1985) describe the isolation of a 65,000 MW protein from bovine striatum using affinity chromatography with b-naltrexylethylenediamine. Maneckjee et al. PNAS USA 82:594-598, 1985) disclose three proteins having MWs of 92,000, 42,000 and 35,000, which were identified from rat brain using affinity chromatography with "Hybromet" a m-selective ligand. Cho et al. (PNAS USA 83:4138-4142, 1986) and Ueda et al. (Neurosci. Lett. 75:339-344, 1987) both teach the isolation of a 58,000 molecular weight species from rat brain by affinity chromatography with 6-succinyl morphine as ligand. The peptide described by Cho et al. has subsequently been shown not to be a transmembrane spanning protein (Schofield et al., EMBO J. 8:489-495, 1989). Simon et al. (Neuropeptides 10:19-28, 1987) describe the isolation of 65,000 and 58,000 MW peptides from frog brain by affinity chromatography with the opioid peptide DADLE. Ahmed et al. disclose a 66,000 MW species which was isolated from human placenta by binding to thiol-sepharose, followed by gel electrophoresis, and binding to wheat germ agglutininagarose. Notwithstanding these many reports, however, none of these species has ever been verified as an opioid receptor, nor has any of them ever been reported to yield either amino acid or nucleotide sequence which was verifiable as encoding a functional receptor. The logical inference is that the "receptors" allegedly purified in these papers were either not adequately pure to permit sequencing, or are not in fact the receptors they were believed to be. A need therefore continues to exist for a verifiable isolated opioid receptor sufficiently pure to allow sequence to be determined. The invention described in the present application now fulfills such a need.

## SUMMARY OF THE INVENTION

The present invention relates to a substantially pure opioid receptor protein, and biologically active fragments thereof. In addition the invention relates to the nucleotide sequence encodes that for the opioid receptor. The term "substantially pure" as used throughout the present specification and claims, means a protein free of other non-opioid receptor cellular proteins with which it would normally be associated in its membrane-bound state. Such a protein is essential in order to successfully obtain accurate sequence information. A purified opioid receptor is isolatable by binding a biotinylated opioid ligand with membranes derived from an appropriate tissue source, i.e., one expected to express opioid receptors, to form a receptor:ligand complex. The membranes are then solubilized in a bile-salt like detergent composition, and contacted with an avidin or streptavidin containing affinity substrate, to which the biotinylated receptor:ligand complex will bind. The receptor is eluted from the bound complex by contact with an eluant containing GTP and NaCl or NaCl alone. The eluate is then contacted with a lectin affinity column which specifically binds glycoproteins.

In one embodiment, a receptor is identified by its binding a b-endorphin ligand. In particular, three species are identifiable by this characteristic in the method described. A primary species has a molecular weight of about 66,000, while two minor species have molecular weights of 140-160,000 and 50-55,000. Based on the affinity for b-endorphin, and other pharmacological data, these species are believed to represent a m opioid receptor type.

In addition to its use in sequencing and ultimate cloning of the receptor gene, the purified receptor, or biologically active fragments thereof, can be used in production of monoclonal or polyclonal anti-receptor antibodies and to identify patterns of post translational modifications and to Elucidate associated G. proteins. "Biologically active" in the present context refers not only to fragments which retain ligand binding activity, but also refers to fragments capable of raising an antibody response when injected into a host animal. Such antibodies (poly- or monoclonal) can be used in manipulation of peripheral opioid receptors involved in gut motility and growth hormone secretion. Such antibodies can also be utilized in drug delivery to specific tissues or for tumor imaging.

Receptor clones isolated utilizing sequence information obtained from the purified protein are useful in identifying other receptor subtypes, in screening for new opioid ligands, and for understanding mechanisms of opioid action, for example, drug addiction.

## BRIEF DESCRIPTION OF THE FIGURES

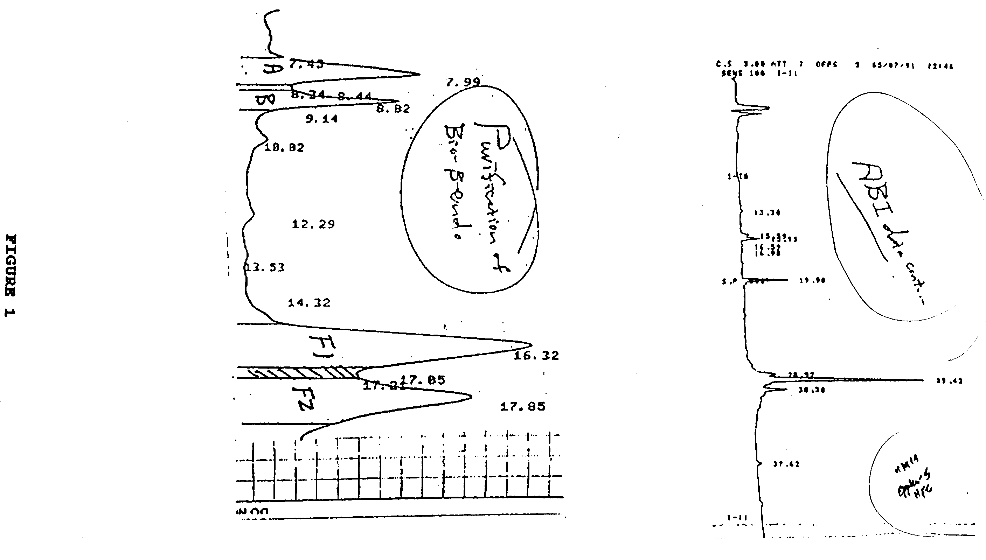

**Figure 1**. **Purification of Biotinylated b-Endorphin**. A. Analytical HPLC of crude biotinyl b-endorphin. B. Preparative HPLC of biotinylated b-endorphin.

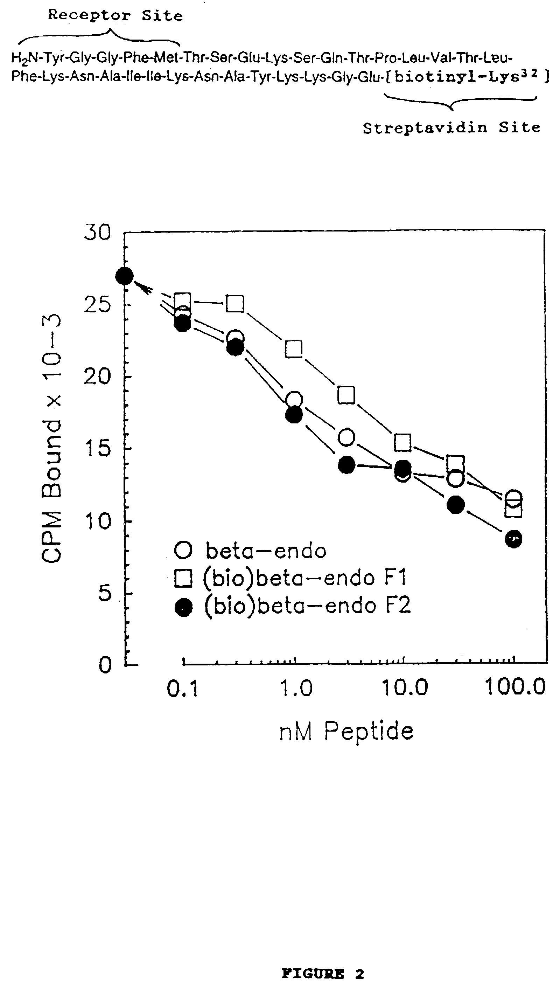

**Figure 2**. **Relative Receptor Binding Activities of Biotinylated and Nonbiotinylated b-endorphins**. Rat brain membranes (30 mg per well) are incubated with [$^{125}$I]b-endorphin (100,000 cpm per well) in the microtiter plate assay as described in Experimental Procedures. 2.A. Binding incubations are for 1 hour.

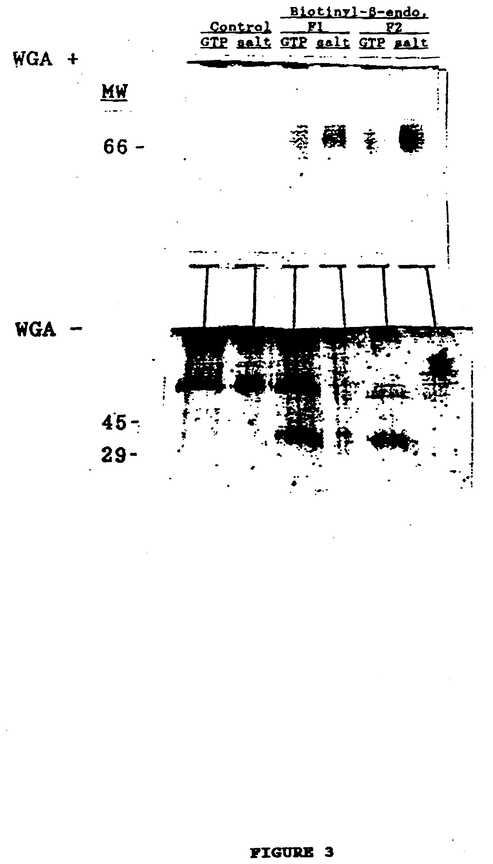

**Figure 3. Efficacy of Biotinyl-b-Endorphin in Receptor Purification.** Rat brain membranes (each containing 30 mg of protein) are incubated with: (a) No ligand, (b) 100 nM biotinyl-b-endorphin F1 or (c) 100 nm biotinyl-b-endorphin F2 and solubilized in 0.15% D:L (all procedures as in Experimental Methods. 1.-3.: One exception in this particular experiment is that the binding and wash step is done in 50 mM potassium phosphate [pH 7.4] + 0.1% BSA). The 100,000 x g supernatants are then incubated with immobilized streptavidin (streptavidin agarose or SA-A) and the SA-A is poured into chromatography columns and washed with solubilization buffer + D:L as described in Experimental Procedures. 6.). Each column is eluted at room temperature, first with 4 column volumes of 100 mM GTP-q-S and then with 4 column volumes of 500 mM NaCl (both eluants in solubilization buffer __ 0.15% D:L + 1/1000 volume of 100X 4Pase. The eluates are then incubated with WGA-agarose and processed for analysis by SDS-PAGE of glycoproteins (proteins bound to WGA-A) and nonglycoproteins (proteins not bound to WGA-A) as described in Experimental Procedures. 6. and 7.

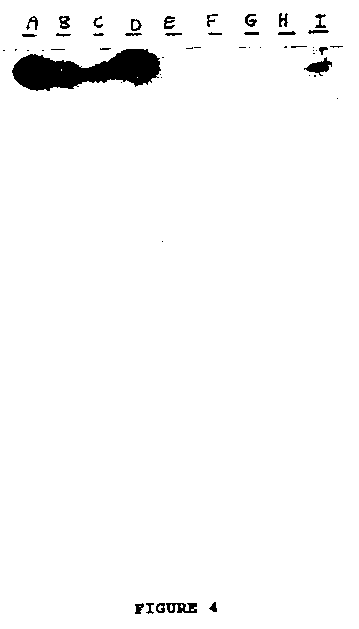

**Figure 4. Immunoreactivity of 30-40,000 MW Material Eluted by GTP from Streptavidin-Agarose Previously Incubated with R:L Complexes Made with Biotinylated Somatostatin or b-Endorphin.** WGA-A nonbound fractions of GTP eluates from streptavidin from receptor purifications done with biotinyl-somatostatin 28 (bio-S28) or biotinyl-b-endorphin are separated by SDS-PAGE and analyzed by Western blotting. The electroblotted samples are first reacted with an anti-$G_{ia}$ rabbit antiserum (1:400 dilution). The second antibody is a peroxidase-coupled, donkey antirabbit Ig antiserum (1:104 dilution). The final complex is detected by enhanced chemiluminscence (ECL; Whitehead, T.P. et al., Nature 305:158-159, 1983). ECL is based on the peroxidase-catalyzed oxidation of luminol and subsequence enhanced chemiluminescence where the probe is bound (Amersham Life Science Products catalog, 1989/90 edition, page 5). Lanes A-C contain 200, 100 and 50 ng of recombinant $G_{ia2}$. Lanes D and E are from a purification of SRIF receptor by the method of Eppler et al. (as described in U.S. Ser. No. 07/677,003) with 20 nM bio-s28, Lane E, binding step done with 20 nM bio-S28 + 20 mM S14. Lanes F-I are from a purification of opioid receptor by the method described herein. F and G are eluates with 500 mM NaCl. H and I are eluates with 100 mM GTP-g-S. F and H are from a sample without ligand in the binding step. G and I are from a sample with 100 nM biotinyl-b-endorphin in the binding step.

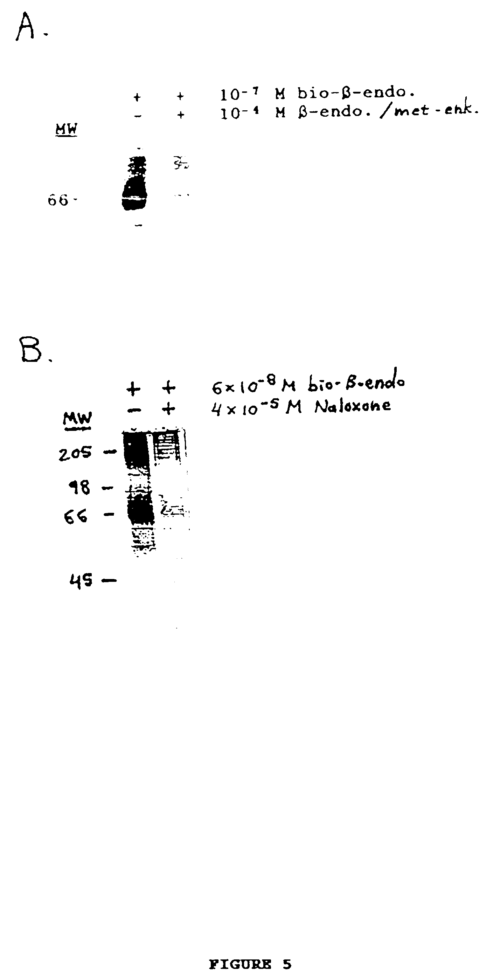

**Figure 5. Effects of Competition by Non-Biotinylated Opioid Ligands on Purification of Receptor Bands by Bio-b-Endorphin.** Competition with biotinyl-b-endorphin in the binding step is by 50 mM b-endorphin + 50 mM met-enkephalin (A) or by 40 mM naloxone (B). In B, 20 mM naloxone is also added to the 100,000 x g supernatant before incubation with streptavidin-agarose because of the relatively high rate of dissociation of naloxone from opioid receptors. Elution from SA-A is with 500 mM NaCl. Eluates are processed by adsorption to WGA, eluted from WGA by TAC and analyzed by SDS-PAGE as described in Experimental Procedures 6.

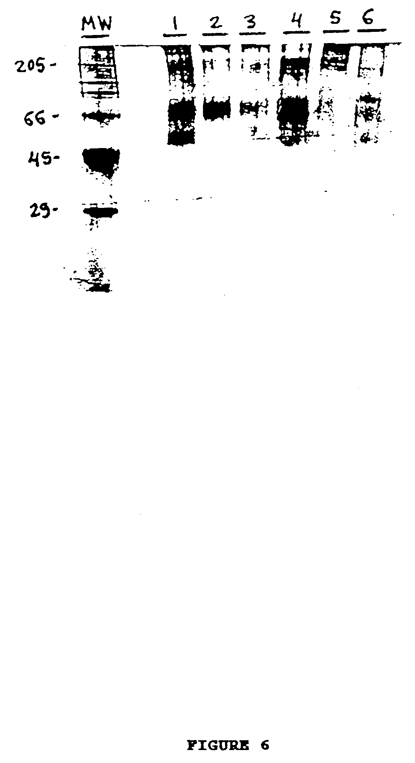

**Figure 6. GTP-Na$^+$ Interactions in Elution of Opioid Receptor from Streptavidin-Agarose. I.** Receptor purification is carried out as described in Experimental Procedures. 6. up to the point of washing the SA-A columns. Two SA-A columns are then eluted in different ways as described in the text. The eluates are processed by adsorption to WGA, elution from WGA with TAC and analysis of the TAC eluates by SDS-PAGE.

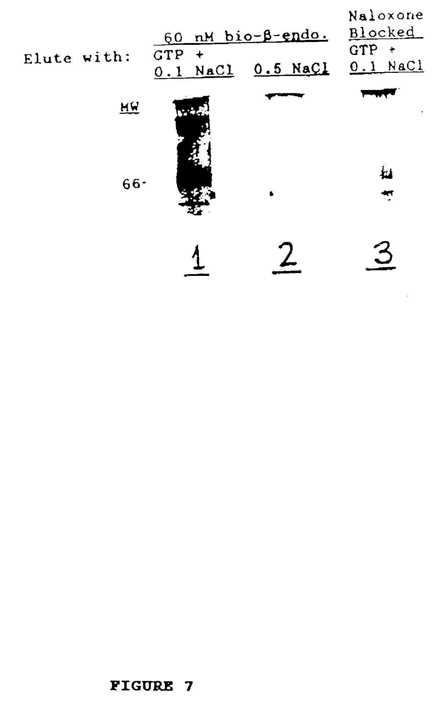

**Figure 7. GTP-Na$^+$ Interactions in Elution of Opioid Receptor from Streptavidin-Agarose. II.** Receptor purification is carried out as described for Figure 6 up to the point of washing the SA-A columns. Elution is by 100 mM GTP-100 mM NaCl (1) and then by 500 mM NaCl (2). Further processing of the eluates is as described for Figure 6.

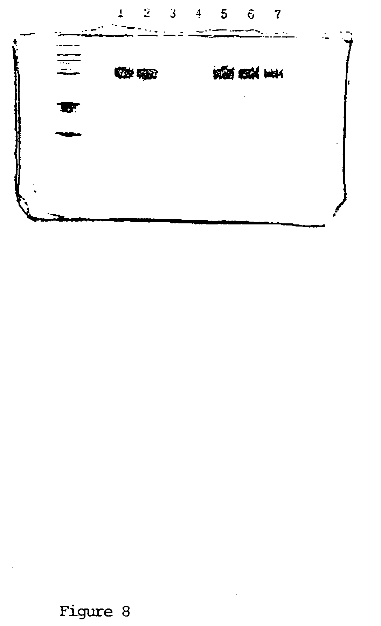

**Figure 8. Competitive Binding of Mu and Delta Receptor-Specific Peptide with b-Endorphin for Binding with Opioid Receptor.** This 11% SDS-PAGE gel illustrates the mu-subtype identity of the isolated receptor protein. Lanes 3 and 4 demonstrate the ability of a peptide having preferential binding with a mu-subtype receptor to competitively inhibit binding of a biotinylated b-endorphin to the receptor, thereby preventing isolation of the 66 kDa protein from rat membranes using the biotin-avidin affinity chromatography. Lanes 5, 6 and 7 represent competitive binding using a delta-subtype specific peptide which permits recovery of the 66 kDa protein.

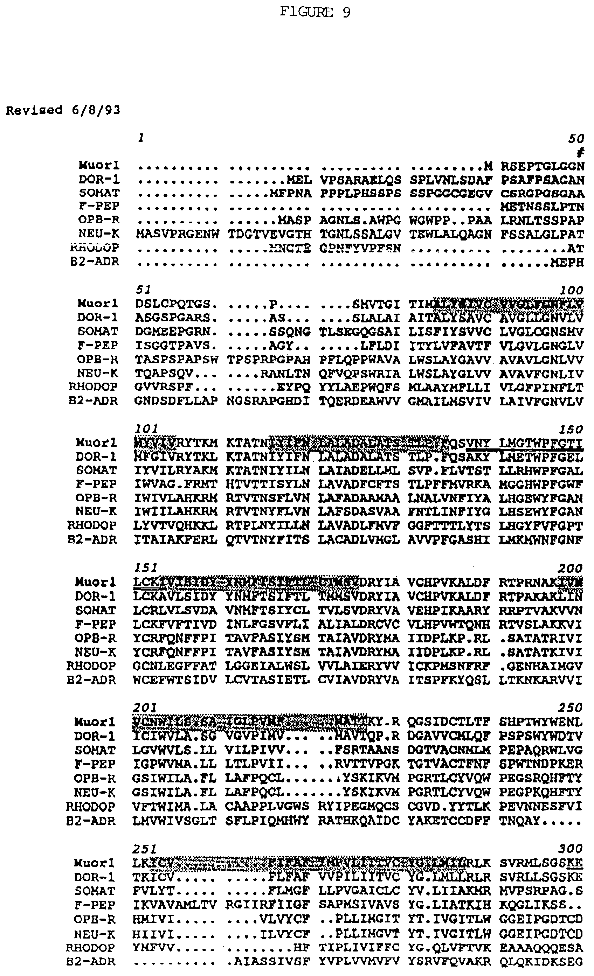

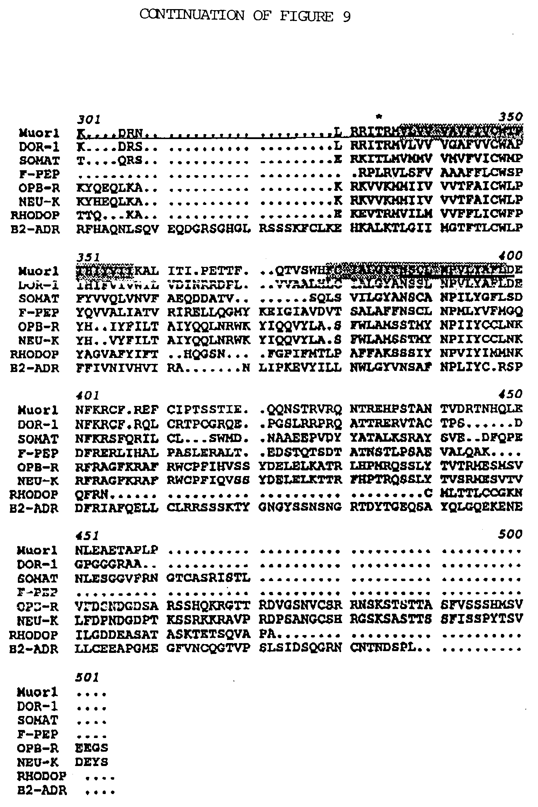

**Figure 9. Amino acid sequence of the rat brain u-opiate receptor predicted from the sequence of the cDNA RC8-I, and comparisons of sequence of other G-linked receptor family members**. RC8-1 is subject to double stranded sequencing by automated and manual means, and the translation product open reading frame aligned with those of the mouse w-opioid receptor (DOR-1), rat somatostain receptor (SOMAT), human N-formylmethlonine receptor (F-PEP), human opioid hinding protein (OPB-R), rat neuromedin K recptor (NEU-K), rat rhodopsin (RHODOP), and rat beta2 adrenergic receptors (B-2ADR).

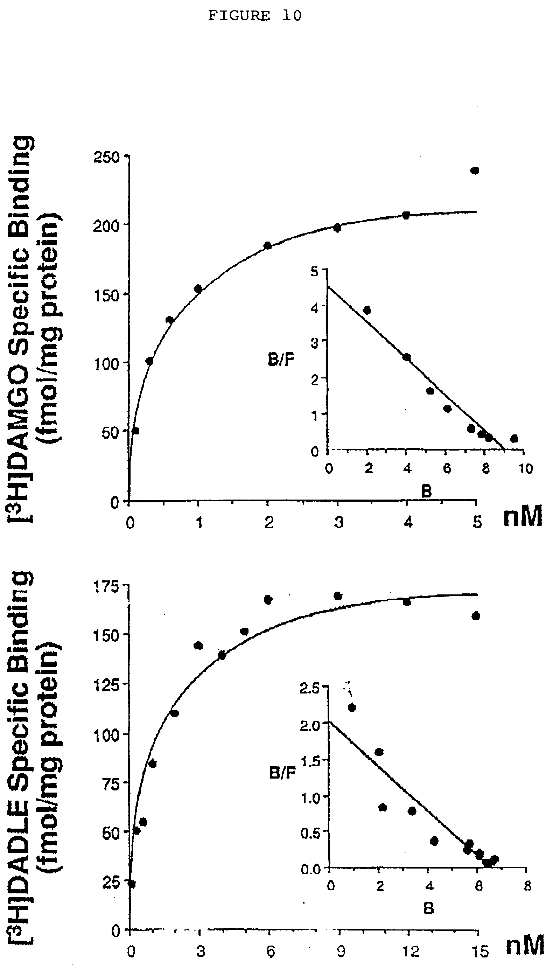

**Figure 10. Saturation analyses of binding of [3H]DAMGO (top) and [3H]DADLE (bottom) of membranes prepared from COS cells transfected three days before assay with pcDNA1RC8-1-1.** Receptor binding is carried out with 100 nM naloxone added to parallel incubations to estimate nonspecific binding.

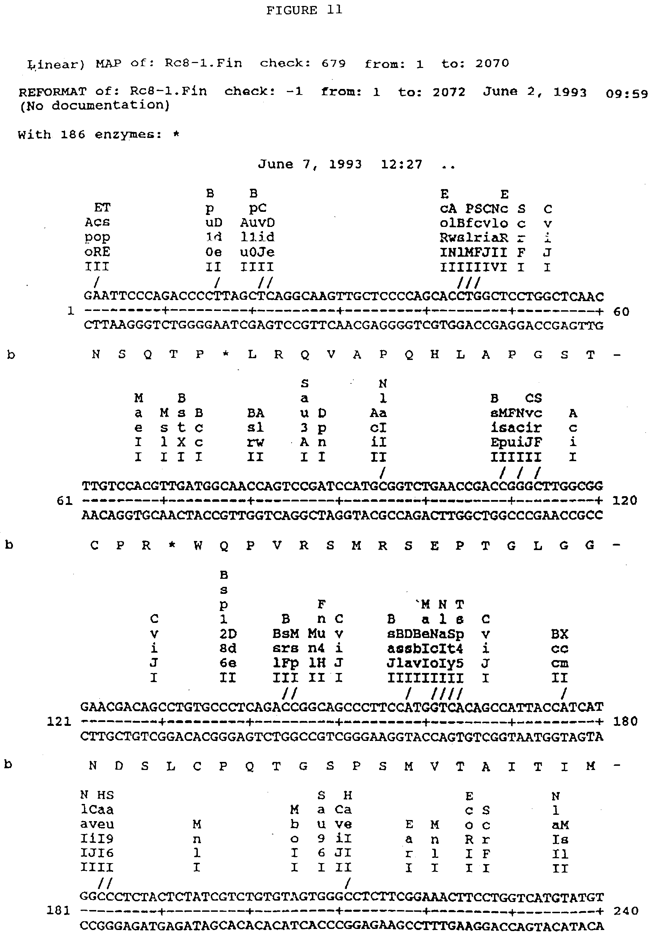

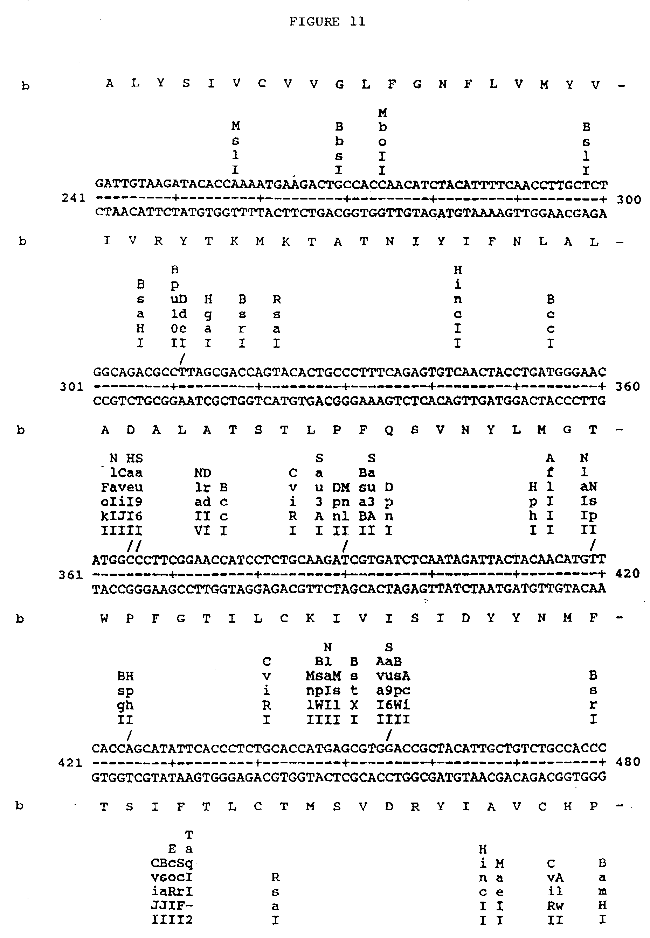

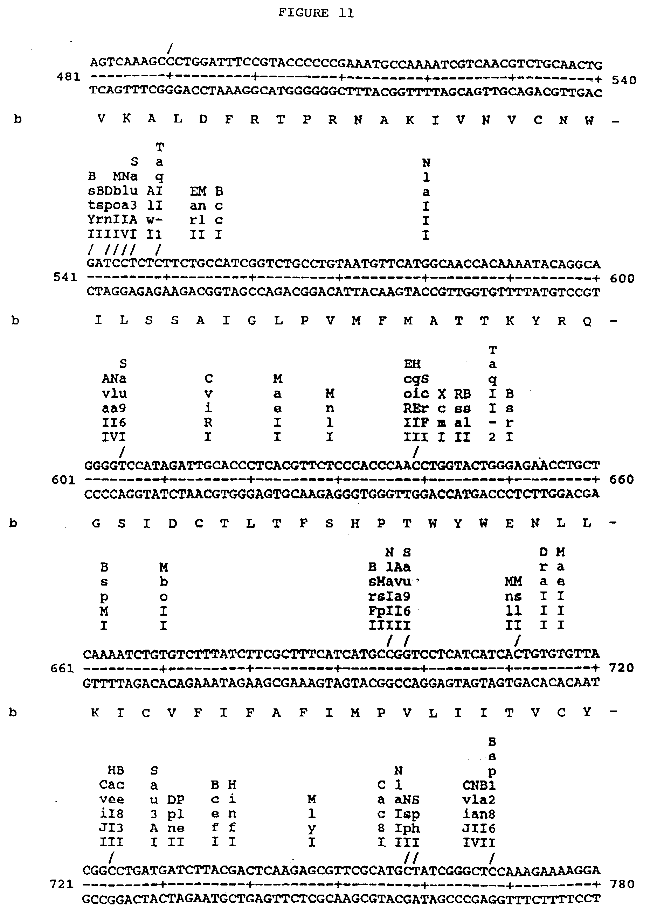

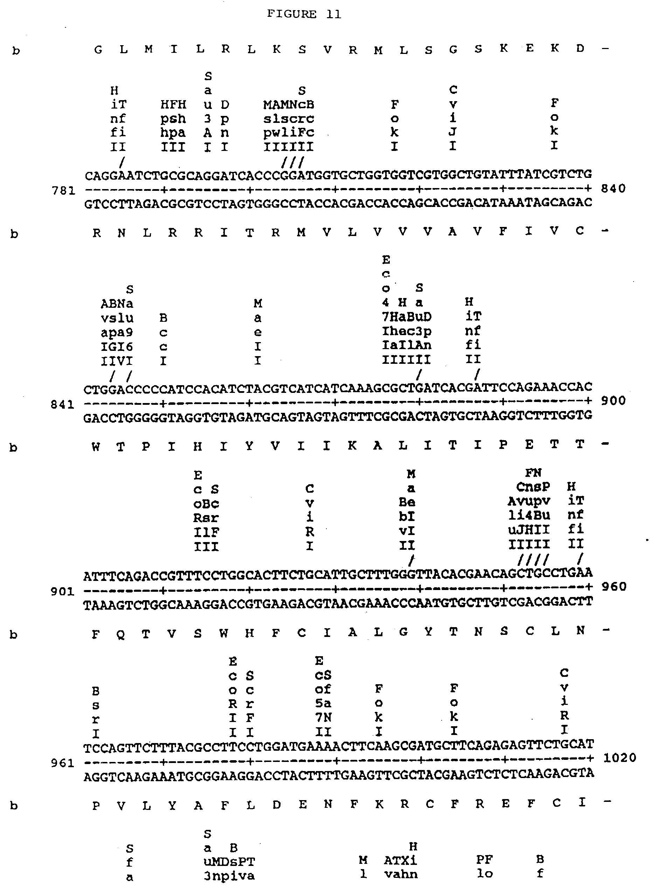

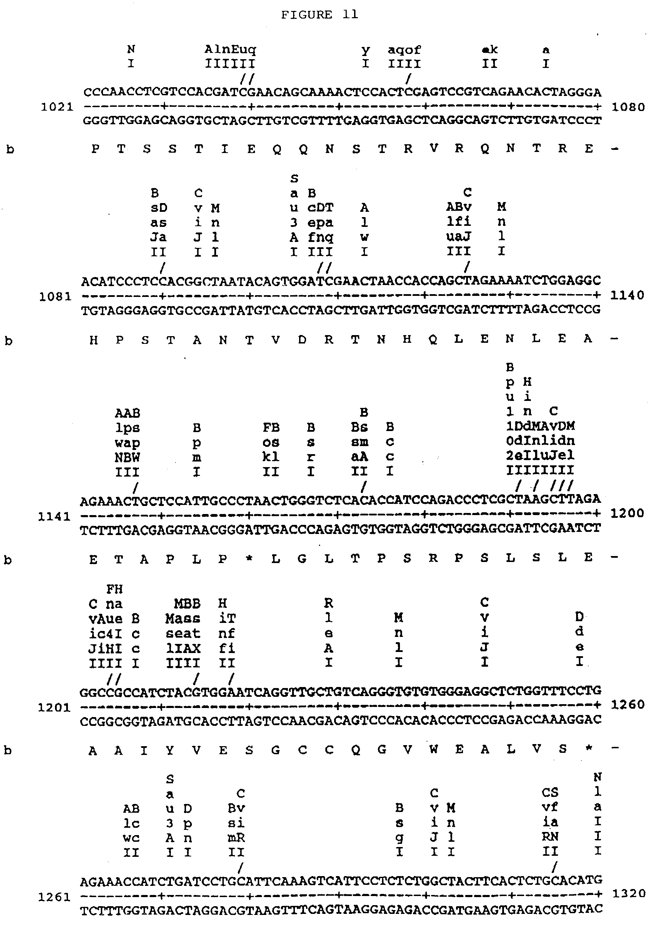

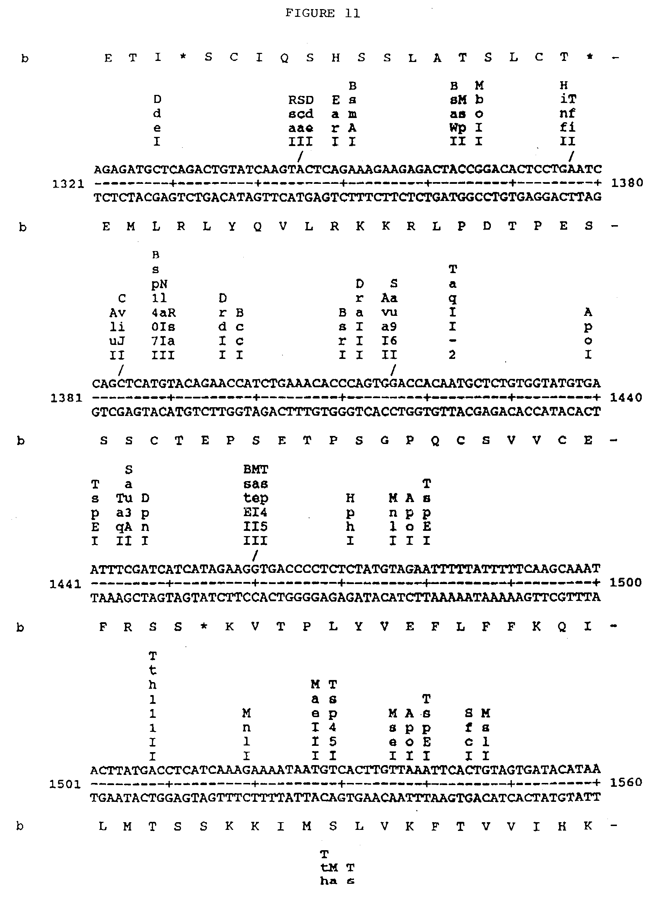

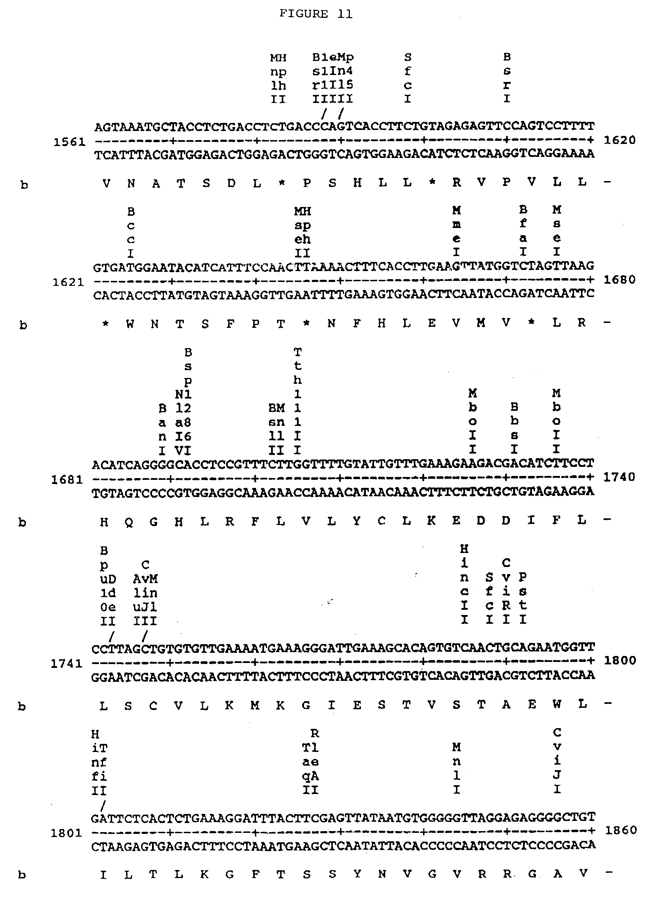

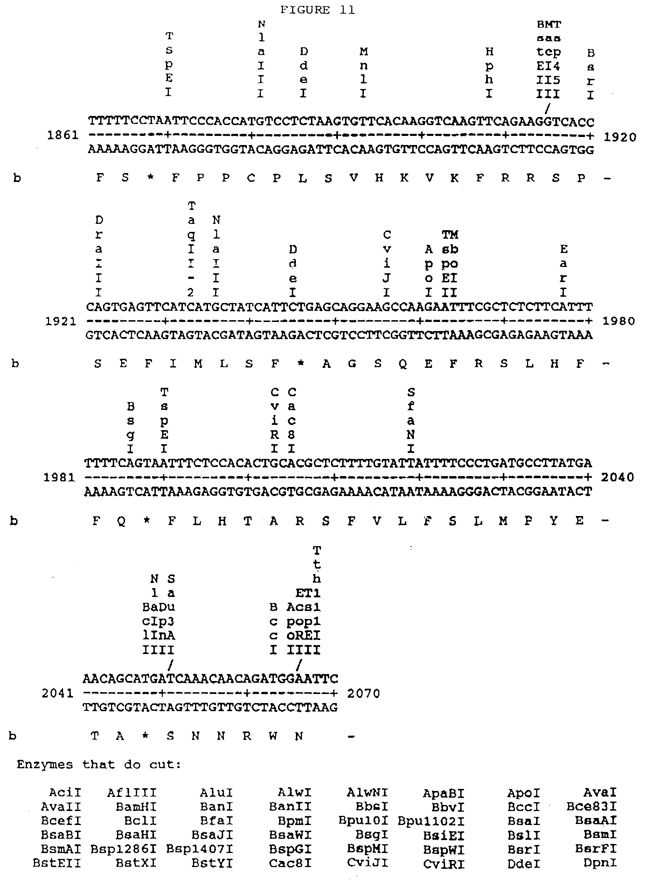

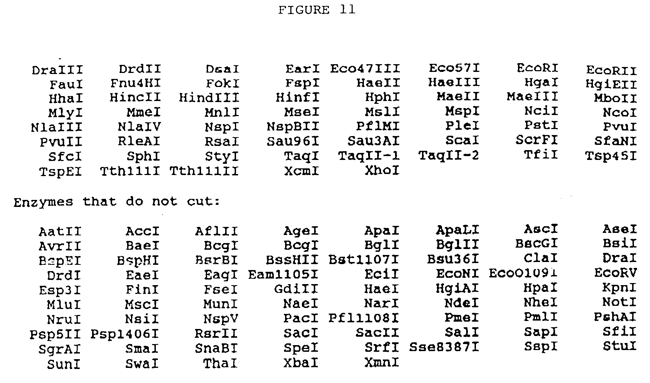

**Figure 11. Nucleotide Sequence of m receptor and predicted amino acid sequence of m opiate receptor C-DNA, open reading frame analyzed for plasmid CDNA.**

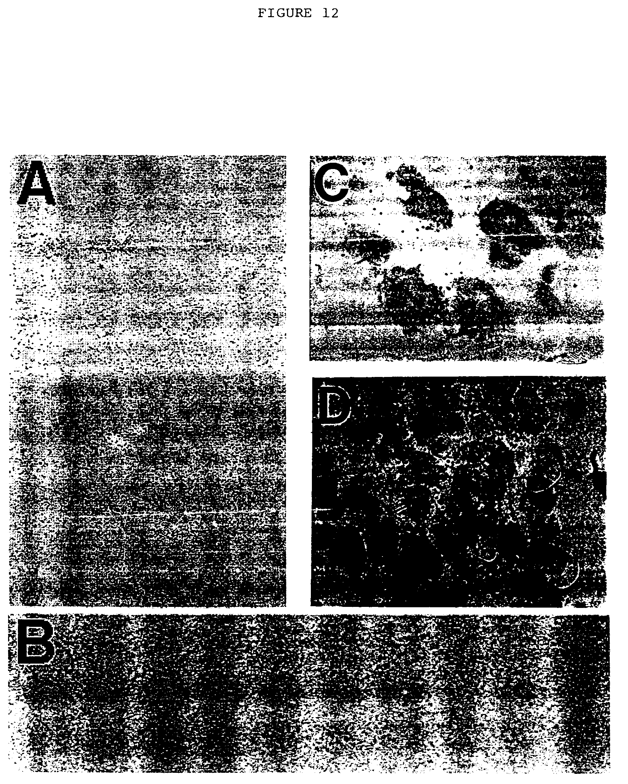

**Figure 12. Expression of m-opiate receptor RC8-1 mRNA.** A. Northern analyses and phosphoimager autoradiogram of m-opiate receptor mRNA hybridization to radiolabeled kRC8-1 cDNA in 20 mg total RNA extracted from rat thalamus (lanes 1 and 2), or hypothaiamus (lane 4). Size markers (lane 3) suggest a 10.5 kb mRNA size.

B. RNase protection and phosphomiager autoradiogram of a 400 basepair fragment protected from digestion by 5 mg of m-opiate receptor mRNA extracted from: (1-r) lane 1: cerebral cortex; lane 2: straitum: lane 3: thalamu; lane 4; hypothalamus; lane 5: midbrain; lane 6: hippocampus; lane 7: brainstorm; lane 8: cerebellum; lane 9: cervical spinal cord; lane 10; liver.

C & D Photomicrographs of emulsion autoradiograms of in situ hybridization grain densities overlaying thalamic (C) and lateral habcnula, medial aspect (D) neurons expressing mOR mRNA in a representative section through rat diencephalon (). Similar results are obtained in experiments using tow distinct mOR mRNA hybridization probes, and in sections from three different brains.

Objective magnification = 100 x (C), and 4 x (D).

## DETAILED DESCRIPTION OF THE INVENTION

The purified-opioid receptors are isolated by a receptor purification method disclosed in U.S. Serial No. 07/677,003, the contents of which are incorporated herein by reference. The purified opioid receptors are described in U.S. Serial No. 07/026,140 the contents which are incorporated herein by reference. Opioid receptors can be found in a wide variety of tissue types (Jaffe and Martin, supra, the contents of which is incorporated herein by reference). In particular, the w, m, k, and s classes of receptors are found in brain, as well as other tissues; the e type is found in vas deferens and the k type is also plentiful in placenta (Ahmed et al., supra).

The opiate receptor is isolated initially as a complex with its associated G proteins. A number of opiate or opioid analogs are commercially available that can be used for receptor binding. For example, Research Biochemicals, Incorporated, 1991 Catalog, page XV, identifies a number of opioid ligands by their subtype specificity. The ligand used will generally be selected based on its affinity for a particular receptor subtype. In a preferred method for purification, a biotinylated opiate analog is used. In the following examples, the ligand used for isolation of receptor is a biotinylated b-endorphin. In the preferred isolation method, the ligand is first bound to intact cell membranes, thereby forming a receptor:ligand (R:L) complex. After this prebinding step, the membranes are solubilized in detergent and intact receptor:ligand complexes are obtained. A useful detergent for this purpose is a combination of deoxycholate and lysophosphatidylcholine in a 1:1 ratio, preferably at a concentration of 0.2% w/v or less. At this stage, the complex consists of the receptor and its associated G protein subunits. The association of the receptor with G proteins is confirmed by the rapid dissociation of the complex in the presence of a stable GTP analog.

The solubilized complex is then contacted with an appropriate high affinity binding column. When the ligand is biotinylated, the column used is preferably streptavidin-agarose (SA-A), whereby the biotinylated portion of the R:L complex will tightly bind to the streptavidin. Streptavidin is preferred, due to its lower non-specific binding; however, free and immobilized avidin is also available (Pierce, Vector) and may be suitable for some purposes. The column is eluted with a GTP analog, such as GTP-q-S. The GTP analog serves to dissociate G protein subunits from the receptor, thereby lowering the affinity of the receptor for its ligand, and thus indirectly causing dissociation from the ligand. In a preferred embodiment, the elution with GTP analog is combined with elution with at least 25 mM NaCl, preferably 50-100 mM, up to a maximum of about 500 mM NaCl. Although dissociation will occur with GTP alone, it occurs at a relatively low level (about 30%), and the use of NaCl enhances this dissociation. Alternately, a high level, i.e., 500 mM of salt can be used alone. The eluate from the streptavidin column is then incubated with a lectin affinity chromatography substrate, such as wheat germ agluttinin (WGA)-agarose, which will separate glycoproteins from nonglycoproteins. The eluate containing the glycosylated material shows a protein with a molecular weight of about 66,000; this protein is also seen in material eluted by GTP-q-S and/or with NaCl, but is not seen in eluates from samples not previously bound with the biotinylated b-endorphin, indicating its ligand dependence. This band appears to represent an opioid receptor, presumably a "mu" or "delta" type opioid receptor, based on b-endorphin's known preferential binding to "mu" or "delta" receptor types, and the pharmacological data discussed below.

In the nonglycosylated material that is not bound to the lectin affinity column, there appears a second smaller band of about 30-40,000, which apparently elutes with GTP-gamma-S alone (i.e., without NaCl). This material also is apparently ligand-dependent, since, like the 66K band, it only appears in eluates from samples which have been prebound with biotinyl-B-endorphin. It is assumed that this band represents G protein subunits, particularly in light of binding with anti-$G_{ia}$ as seen in Figure 4.

The identity of the isolated material is confirmed by additional experiments using nonbiotinylated b-endorphin analog ligands. A [125I]b-endorphin analog is used as described above to create R:L complexes in rat brain cell membranes. These complexes, when applied to wheat germ agluttinin, and eluted with N-N'-N''-triacetyl-chitotriose, shows a fairly high level of specifically bound material (see Tables 1 and 2, infra), confirming the identity as a glycoprotein.

The purified 66 kDa glycoprotein is subjected to Lys-C endoprotease digestion, SDS polyacrylamide gel electrophoresis and electroblotting, producing a 15 kDa peptide band. This peptide yields 20 cycles of high quality amino acid sequence. The N-terminus of this band overlaps by 4 amino acid residues with a 7-amino acid residue sequence obtained from a bond of about 3 kDa from a cyanogen bromide digest, giving a total sequence length of 23 amino acid residues. The sequence (Sequence ID No. 1) obtained is as follows:

**Lys-Glu-Lys-Asp-Arg-Asn-Leu-Arg-Arg-Ile-Thr-**
**Arg-Met-Val-Leu-Val-Val-Val-Ala-Val-Phe-Ile-**
**Val**

This sequence is quite similar to a region of the SSTR1 somatostatin receptor, spanning parts of intracellular loop III and transmembrane region VI. Significantly, it is 83% identical with the same region of a recently cloned delta opioid receptor from mouse (C.J. Evans, et al., Science 258:1952-1955, 1992). Underlined residues indicate the differences between the two receptors in this region.

Pharmacological evaluation of the purified protein indicates that it is a mu-subtype receptor, and that the difference between the repacted delta subtype receptor and the present receptor is not attributable to a simple species difference, but is due to a known mu-specific peptide capable of blocking the binding of b-endorphin to the isolated receptor.

The novel sequence information obtained provides the basis for isolation and cloning of the corresponding gene encoding the receptor. The delta opioid sequence in this region is nearly identical to the same region of SSTR1, and seems to be highly conserved in a set of 5 or 6 receptors, indicating homology in the mu receptor as well. The combination of primers, including the mu specific-based primer, in PCR of whole brain mRNA, selectively yields the mu receptor. Sequencing the CDNA's yields clones whose sequencing predict the same amino acids found in the purified opiate receptor preparation expression of there C-DNA, yield m opiate receptor binding.

The purified receptor, or biologically active fragments thereof, are useful for a number of purposes. For example, the purified material, in glycosylated or nonglycosylated form, is used to create monoclonal or polyclonal antibodies having specificity for the opioid receptor. The technology for creation of monoclonal antibodies is well known in the art (see, e.g., Goding, Monoclonal Antibodies: Principle and Practice, 2nd Ed., 1986). Such antibodies have utility in manipulating purified opioid receptors involved in gut motility and growth hormone secretion, or in drug delivery to specific tissues or for tumor imaging. General techniques for preparing anti-receptor antibodies are found in U.S. Patent No. 4,857,637, the contents of which are incorporated herein by reference.

The isolated receptor protein itself, and protein expressed from the cloned opiate receptor CDNA is useful in screening assays to identify compounds that act as analogs. For example, the receptor protein is immobilized by any means which does not interfere with opiate binding activity. The immobilized receptor is then contacted with a specific compound or mixture and its ability to compete with radiolabelled opiate for binding to the receptor is evaluated. Variations on this method are apparent to those skilled in the art.

The present invention encompasses the opiate receptor protein and its biologically active fragments produced by any means, whether synthetically, recombinantly, or by purification of the native protein. The isolated opiate receptor, as described above, is used in protein sequencing procedures. The protein sequence in turn is used to design oligonucleotide probes used to screen ggt10 libraries containing the relevant cDNA (copies of RNA), e.g., from brain cells. Hybridization of oligos with the library identifies the clone(s) containing the SRIF receptor gene or portions thereof. The gene or gene fragments are isolated from the clones, the whole gene reconstructed and then ligated into an appropriate vector by known methods. The vector is chosen based upon the choice of preferred host cell. The host cell is prokaryotic, e.g., E. coli or other bacteria; or eukaryotic, e.g., yeast, insect, or mammalian cells.

The following non-limiting examples further illustrate the present invention.

## EXAMPLES

### I. EXPERIMENTAL PROCEDURES

The following materials and methods are referred to throughout the Examples.

## 1. Preparation of Rat Brain Membranes

Whole male rat brains frozen in liquid $N_2$ are purchased from Pel-Freez (Rogers, AR). All procedures for membrane preparation are carried out at a temperature of 2-6°C. The brains are homogenized in a Waring blender in a buffer containing 1 mM Na-bicarbonate (pH 7.2), 1 mM EDTA, 1 mM EGTA (all chemicals from Sigma Chemical, St. Louis, MO) and 0.7% (vol./vol.) of the 100X 4Pase protease inhibitor mixture (see "Protease Inhibitors" below). The ratio of tissue/homogenization medium is from 25-35 gm of brain/500 ml. The blender is controlled through a variable output rheostat (Staco Energy Products, Dayton, OH; type 3PN1010) at a setting of 40. The homogenate is centrifuged for 10 minutes at 1,000 x g pellet is rehomeginized in 500 ml of homogenization medium and recentrifuged for 10 minutes at 1,000 x g. The 1,000 x g pellet is discarded. The 1,000 x g supernatants are combined and centrifuged for 30 minutes at 20,000 x g. The 20,000 x g membrane pellet is washed by being resuspended with a Dounce homogenizer in 500 ml of homogenization medium supplemented with 10 mM EDTA (pH readjusted to 7.4) and then washed twice by being resuspended in 25 mM Tris buffer (Sigma Chemical Co.; pH 7.4) and centrifuged for 25 minutes at 20,000 x g. The final membrane pellet is resuspended in 25 mM inhibitor mixture to a protein concentration of 4-12 mg/ml. The resuspended membranes are aliquoted, frozen on dry ice and stored at -90°C.

## 2. Receptor Binding Methods

Binding of [$^{125}$I]-labelled b-endorphin, b-endorphin and other b-endorphin analogs and opioids is done in a binding buffer containing 50 mM HEPES (Sigma; pH 7.4; pHed with KOH), 0.1% (w/v) bovine serum albumin (Miles Laboratories, Elkhart, IN) and protease inhibitors as specified below for specific applications. All binding incubations are carried out at room temperature (20-23°C).

A. Analytical - This assay is carried out in 96 well microtiter plates (Immulon II with snap-off wells; Dynatech, Chantille, VA). To carry out the assay, the following components are added to the wells in the order and volumes shown: (1) 5 ml of nonlabelled ligand. For this purpose, ligands (for example b-endorphin or biotinylated b-endorphin) are made up at 40X the desired final concentration in the 40X P/B/Pz protease inhibitor mixture (Experimental Procedures. 4.C.) (2) 50 ml of [$^{125}$I]b-endorphin in binding buffer + 1/100 volume of the 100X 4Pase protease inhibitor mixture; mix briefly on a microtiter plate shaker (Dynatech Micro-Shaker II, Dynatech, Chantilly, VA). (3) 145 ml of rat brain membrane diluted in binding buffer + 1/1000 volume of 100X 4Pase to deliver 30-50 mg of membrane protein per well. The plates are then covered with Linbro Mylar plate sealers (Flow Labs, McLean, VA) and incubated for 1 hour at room temperature (20-23°C). The membranes are pelleted by centrifugation at 2,000 x g, the supernatants (containing nonbound ligands) are decanted and the pellets are washed by the addition of 200 ml of ice cold binding buffer, brief shaking and recentrifugation. CPM of [$^{125}$I] in the final membrane pellets is then counted in a gamma counter (LKB Gammamaster 1277; 80% efficiency).

B. Preparative - Rat brain membranes are diluted to a concentration of 0.5 mg of membrane protein/ml in binding buffer containing 1/400 (vol./vol.) of the 400X P/B/Bz protease inhibitor mixture (see Section 4). Biotinyl-b-endorphin (synthesized and purified as described below; 1:1 mixture of HPLC fractions 1 and 2 is added, most commonly to a concentration of 60 nM. The mixture is incubated either by stirring in a large polypropylene beaker (1-2 liters volume) or by rotation on a tube rotator (100-250 ml per polypropylene centrifuge tube). Control incubations designed to show ligand specificity of purified proteins are done by various means as follows: **i. No ligand.** Rat brain membranes are incubated as above except with no biotinyl-b-endorphin or other opioid analog. **ii. Blocking ligand.** Binding of biotinyl-b-endorphin is blocked by a large molar excess (500-1,000 fold) of a non-biotinylated opioid ligand such as b-endorphin , met-enkephalin or naloxone. In this case, the blocking ligand is added from 5-15 minutes prior to the addition of biotinyl-b-endorphin. In some cases only the blocking ligand is added. For example, the receptor sites may simply be saturated with naloxone. The binding reactions (1 hour) are terminated by centrifugation for 10-15 minutes at 20,000 x g. The supernatants are decanted and the membrane pellets are washed with a volume of binding buffer (minus bovine serum albumin) equal to the original incubation volume. For this wash step, the membranes are dispersed in the wash buffer in a Dounce homogenizer, diluted out in the wash buffer and recentrifuged at 20,000 x g. This final membrane pellet is then solubilized in detergent as described in part 3, below, and used to characterize soluble R:L complex (when prebinding is done with [$^{125}$I]b-endorphin) or for purification of opioid receptor and associated G protein (when prebinding is done with biotinyl-b-endorphin).

### 3. Solubilization of B-Endorphin:Opioid Receptor Complexes (R:L Complexes)

This step is carried out in a solubilization buffer containing 25 mM Tris (pH 8.0) and 10% glycerol. All procedures are at 4°C or on ice. Deoxycholate:lysophosphatidylcholine (1:1 (w/w) mixture; hereafter referred to as D:L; stock solutions = 10% (w/v) in $H_2O$; purchased from Sigma] is added to the solubilization buffer to a final concentration of 0.15% w/v (deoxycholate = 0.075%; lysophosphatidylcholines = 0.075%). Protease inhibitors (100X 4Pase; 1% vol./vol.) are added and rat brain membranes are diluted out into this medium to a protein concentration of 0.5 mg/ml. After 30-45 minutes incubation on ice, the samples are centrifuged for 30 minutes at 100,000 x g. The 100,000 x g supernatants are aspirated out of the centrifuge tubes as far as possible without disturbing the pellets of insoluble material. Then the remaining supernatant is poured out of the tubes and filtered through a 0.2 m cellulose acetate or nylon filter unit (Corning Inc., Corning, NY) to remove particulate matter dislodged from the pellet. This filtered supernatant is then combined with the material removed by aspiration.

### 4. Protease Inhibitors

Three mixtures of protease inhibitors are used in these procedures. **(A) 100X 4Pase.** 5 mg pepstatin A, 15 mg chymostatin, 38 mg leupeptin and 73 mg phenylmethylsulfonylfluoride (PMSF; all compounds from Bachem, Torrance, CA) are dissolved per 5 ml of dimethylsulfoxide (DMSO; Aldrich Chemicals). Aliquots are stored frozen at $4_{°C}$. **(B) 40X PMSF/Baci.** 2 mg of PMSF and 2 mg of bacitracin (Sigma) are dissolved per ml of DMSO. Aliquots are stored frozen. **C. 400X P/B/Bz.** 20 mg of PMSF, 20 mg of bacitracin and 20 mg of benzamidine (Sigma) are dissolved per ml of DMSO. Aliquots are stored frozen.

### 5. Synthesis and Purification of Biotinyl-b-Endorphin

A peptide with the amino acid sequence $H_2$N-Tyr-Gly-Gly-Phe-Met-Thr-Ser-Glu-Lys-Ser-Gln-Thr-Pro-Leu-Val-Thr-Leu-Phe-Lys-Asn-Ala-Ile-Ile-Lys-Asn-Ala-Tyr-Lys-Lys-Gly-Glu--[biotinyl-Lys[32]]-CO ([biotinyl-Lys[32]]b-endorphin) (Sequence ID No. 2) is synthesized at Applied Biosystems Inc. (ABI; Foster City, CA). Synthesis is by solid phase from the C terminal by the Fmoc method. After synthesis, while the peptide is still on the resin, the e-amino group of Lys[32] as specifically deprotected. The e-amino group is then reacted with N-hydroxy-succinimidyl-aminohexanoyl- biotin (NHS-LC-Biotin; Pierce, Rockford, IL; "LC" = aminohexanoate). After biotinylation, all of the protected amino acid residues are deprotected and the peptide is released from the resin by HF cleavage. In the example shown the final product (about 50% pure; see Figure 1A), is further purified by reverse phase HPLC on a Brownlee "Aquapore" C8 column (1 x 25 cm). Elution is by a gradient of acetonitrile mixed in water/0.1% trifluoroacetic acid. Two closely spaced product peaks are eluted from the column. These two peptide fractions are lyophilized and solubilized in water at 1 mg/ml. Aliquots are stored frozen at -90°C.

### 6. Purification of Opioid Receptor

100,000 x g supernatants from rat brain membranes carried through the ligand binding and solubilization steps are incubated with immobilized streptavidin (streptavidin-agarose or SA-A; Pierce Chemical, Rockford, IL). The incubations contain 1 volume of SA-A per 29 volumes of supernatant. Incubations are for 4 hours at 4-8°C on a tube rotator. Then the mixtures are poured into glass chromatography columns (Econo-Columns, Bio Rad Labs, Richmond, CA) and the non-bound material is filtered through the bed on packed resin. The resin is washed with 20 bed volumes of solubilization buffer + 0.15% D:L + 1/500 volume of the 100X 4Pase protease inhibitor mixture. The eluates from the SA-A columns are incubated overnight (12-15 hours) with 1/200 to 1/400 volumes of immobilized wheat germ agglutinin (WGA-agarose or WGA-A; Vector Labs, Burlingame, CA). The WGA-A is pelleted by centrifugation, washed twice with 50-100 volumes of solubilization buffer + 0.15% D:L (after removing the supernatants containing material not bound to WGA) and then either: (A) eluted with 8 mM triacetylchitotriose (TAC; Sigma) in solubilization buffer + 0.15% D:L (3 sequential elutions where resin is mixed with 2 volumes of elution buffer at room temperature for 15-20 minutes, pelleted by centrifugation and supernatant removed and saved) or B. solubilized directly by addition of 1X Laemmli sample buffer and heating at 90°C for 10-15 minutes. These samples are analyzed by SDS-PAGE and silver staining. The nonbound supernatants from the WGA-binding step are concentrated, solubilized in 1X Laemmli sample buffer and analyzed by SDS-PAGE and silver staining.

7. Cloning candiate Rat Brain Opioid Receptor cDNAs:

Candidate partial-length rat brain opioid receptor cDNAs are obtained using several mRNA and cDNA sources and several oligonucleotide primers for PCR amplification. pPCR4A is a 700 basepair (bp) pPCRII (In Vitrogen) subclone of a partial cDNA amplified from single strand cDNA prepared from whole rat brain using 35 cycles at 94°C for 1 minute, 55°C for 1 minute and 72°C for 1 min and 20-base oligonucleotide (5' AGA CCG CCA CCA ACA TAT AC3') and (5' GCT TGA AGT TCT CGT CCA GG3') that are complementary to mouse =-opioid receptor sequences. Sequence analysis, following manual sequencing, reveals an open reading frame predicting amino acids similar to those of the murine =opioid receptor, and identical to 23 amino acids sequenced from opiate receptor protein preparations.

The 700 bp insert from pPCR-4A is excised with EcoRI, radiolabelled by random priming to specific activities of $10^9$ dpm/mg, and used to screen 5 x $10^6$ plaques from a oligo dT-primed rat cerebral cortex lambda ZAP cDNA library that was selected so that inserts are > 1.5kb in size (p6 = 26). Hybridization is performed at 30°C in buffer contained 29% formamide and 6 x SSC, washing is at 50°C in 0.4 x SSC/0.1% SDS, and 2 days' autoradiographic exposure is used. Plasmids are autoexcised from g-Zap phage DNA grown from positive plaques as described (p6 = 26) and analyzed by restriction analyses and DNA sequencing. One clone, termed RC8-1, is subjected to complete sequencing of both strands using automated and manual methods, and subcloned into pcDNA1 (InVitrogen) to yield pcDNA1RC8-1. DNA sequences are analyzed using conventional methods.

8. Characterizing RC8-1 as a m-opiate receptor (mOR) cDNA;

COS cells are transfected by electroporation with 20 ug/$10^7$ cells pcDNA1RC8-1-1 or pcDNA1 vector. Transfected COS cells are plated in DMEM containing 10% FBS, cultured for 2-3 days, and tested for opiate receptor expression by radioligand binding.

For typical radioligand binding assays, medium is removed from 150 mm plates containing 5 x $10^6$ COS cells, plates are rinsed briefly with 50 mM Tris buffer (pH 7.4), and cells are harvested by scraping. Membranes are prepared at 4°C by polytron homogenization in Tris buffer, discarding material pelleted by 15 min 1000 x g centrifugation, and retaining membrance fractions pelleted by a second centrifugation for 30 min at 46,000 x g. Membrane fraction protein concentrations are determined by the Bradford method (Biorad). Membranes from $10^6$ COS cells, corresponding to 50 ug protein, are resuspended in 0.5 ml Tris buffer with various labelled and unlabelled compounds and incubated for 90 min at 22°C. Binding is terminated by filtration through GFB filters (Whatman) and 3 washes with 4 ml Tris buffer at 4°C using a Brandel filtration device. In some assays, NaCl, MgSO$_4$, GTP to ATP are added to incubations (P8-10 = 29-31). Radioligands include [H]DAMGO [D-Ala2,N- methyl-Phc4,Glyo15] enkephalin; 60 Ci/mmole, Amersham), [H]DPDPEpCl [D-Pen2,4'-Cl-Phe4,D-Pen5]enkephalin; 51 Ci/mmole, NEN), [H]DALE (D-Ala2,D-Leu5 enkephalin: 37 Ci/mmole, NEN), and [H]U-69,593 (57 Ci/mmole Amersham). Radioactivity is assesed in a Beckman liquid scintillation counter at 40 efficiency.

9. mOR expression:

RNA is prepared from rat tissues that are rapidly dissected and frozen at -70°C. 20 mg of total RNA is prepared and electrophoresed along with molecular weight standards (BRL) and transferred to nylon membranes. Blots are hybridized with the 2.2 kb [$^{32}$P]-random-primed insert of pcDNA1RC8-1 in 5 x SSPE/1% SDS/150% formamide/2.5 x Denhardt/200 mg/ml herring sperm DNA at 42°C overnight, washed twice in 0.4x SSC/0.5% SDS for 30 min at 52°C, and radioactive patterns identified using a phosphoimaging molecular dynamics device following overnight exposures.

RNase protection assays use incubation of 5 mg of RNA and a [$^{32}$P]-labeled cRNA transcribed from the T7 promoter of pPCR4A linearized with BamH1 to form a 400 basepair fragment, under conditions described by the manufacturer (V1 = 40), with detection of protected fragments using a phosphoimaging system.

For in situ hybridization, 10mm cryostat sections through the diencephalons of rats perfused with PLPG (0.5% paraformaldehyde, 1% glutaraldehyde, 75 mM lyside HC1, 37.5mM Na$_2$HPO pH7.4 and 10mM sodium periodate) (W = 41) are thaw mounted onto slides pretreated with Denhardt's solution, 0.02% Ficoll, 0.02% polyvinylpyrrolidone, 0.02% bovine serum albumin, poly-D-lysine coating and acetylation, and are hybridized with 40- and 50-base $^{35}$S-labelled oligonucleotide hybridization probes that are complementary to bases enconding amino acids indicated in Fig 1 and labelled by a primer extension method to ca.2 x 10 Ci/mMole and gel-purified as described (W = 41). Hybridization at 37°Covernight in a complexbuffer is followed by washing at 50ºC and emulsion autoradiography with 2 week exposures, emulsion development, tissue section staining, and analyses. Grain densities overlying individual neurons are counted and analyzed. with positively-hydridizing neurons identified as those with densities more than five times background autoradiographs values. Neurons are identified based on size, shape, nuclear profiles, and frequent presence of nucleoli.

10. Characterization of Soluble Complexes [$^{125}$I]b-endorphin and Opioid Receptor

An initial attempt is made to determine whether opioid receptors can be manipulated in the same general ways as somatostatin receptors (U.S. Serial Nos. 07/677,003 and 07/677,009). The first experiment is conducted to observe whether radioligand binding to membranes followed by detergent solubilization produce R:L complexes that are stable enough for purification and yet readily dissociable under relatively mild conditions (i.e., GTP, salt, pH changes, etc.). To determine this, [$^{125}$I]b-endorphin is bound to rat brain membranes and solubilized membranes are assayed for the presence of intact complexes between [$^{125}$I]b-endorphin and receptor (R:L complex).

The assay for R:L complex exploits the well known glycoprotein nature of receptors which, like most cell surface proteins, contain covalently linked carbohydrate. The ligand, b-endorphin, is not glycosylated and will not bind to a carbohydrate-binding lectin, such as wheat germ agglutinin (WGA). Binding of the radioligand, solubilized after the binding step, to immobilized WGA is considered to reflect binding of the R:L complex to WGA via oligosaccharide groups on the receptor.

Because opioid receptors appear to be coupled to G proteins, dissociation of the R:L complex by a GTP analog is tested. This is done by incubating the solubilized preparations with GTP-q-S prior to incubation with WGA-agarose. The effects of high salt concentrations and, separately, of low salt concentrations in combination with GTP-q-S on R:L complex dissociation are also tested. The results are shown in Tables 1 and 2 below:

Table 1. **Binding of Solubilized [$^{125}$I]b-Endorphin: Opioid Receptor Complex to WGA-Agarose and Dissociation of the Complex by GTP-g-S and NaCl.** I. Rat brain membranes are incubated with [$^{125}$I]b-endorphin as previously described. The "total" binding sample is incubated with only [$^{125}$I]b-endorphin. The "nonspecific" binding sample is incubated with [$^{125}$I]b-endorphin plus $10^{-6}$ M nonlabelled b-endorphin. After the binding step, the membranes are solubilized as described previously. CPM of [$^{125}$I]b-endorphin in the 100,000 x g supernatant are counted as described previously. Some samples are held on ice as an internal standard for the ratio of total to nonspecific cpm in the starting material ("A. 100,000 x g supernatant"; see "A/B" ratio). Other samples ("B. Supernatants + WGA-agarose") are warmed to room temperature and then receive 100 mM GTP-g-S (Sigma; diluted from a 2 mM stock solution in $H_2O$), 500 mM NaCl (diluted from a 5 M stock solution in $H_2O$) or no treatment and are further incubated for 10 minutes at room temperature. Then all samples are placed on ice, mixed with 60 ml of immobilized wheat germ agglutinin (WGA-agarose; Vector Labs, Burlingame, CA) and incubated for 2 hours at 4-8º on a tube rotator. Then the WGA-agarose is pelleted by centrifugation, the supernatants are removed and the WGA-agarose is washed once in solubilization buffer + 0.15% D:L and counted for radioactivity.

## TABLE 1

|  | A. Total | B. Non-Specific | % Decrease in A/G |  |
|---|---|---|---|---|
| Samples |  |  |  |  |
| R:L Complex |  |  |  |  |
| A. 100,000 x g Supernatant# | -- | -- | 6 | -- |
| B. Supernatants + WGA-agarose... |  |  |  |  |
| Untreated | 16,602 | 249 | 67 | -- |
| GTP (100 mM GTP-g-S) | 10,981 | 213 | 52 | 34% |
| 500 mM NaCl | 2,092 | 172 | 12 | 88% |

Notes:

# This sample is not incubated with WGA-agarose. Total cpm per 280 ul is 37,461 (total) and 6,326 (nonspecific).

* Specific cpm bound to WGA-agarose from the untreated sample is considered to be in intact R:L complex.

Table 2. Binding of solubilized [125I]b-endorphin: opioid receptor complex to WGA-agarose and dissociation of the complex by GTP-g-S and NaCl. II. All steps are done essentially as in Table 1 above except that here GTP-q-S is tested either alone or in the presence of different concentrations of NaCl. Samples of 1.5 ml volume are mixed with 0.35 ml of WGA-agarose

Table 2

| CPM of [125I]b-Endorphin Bound to WGA-Agarose | | | | |
|---|---|---|---|---|
| Sample | A. Total | B. Non-Specific | % Decrease in | |
|  |  |  | A/B | R:L Complex |
| Untreated | 115,219 | 1,823 | 63 | -- |
| GTP (100 mM GTP-g-S) | 88,479 | 1,823 | 48 | 24% |
| GTP + 25 mM NaCl | 28,292 | 1,609 | 17 | 77% |
| GTP + 50 mM NaCl | 13,340 | 1,517 | 9 | 90% |
| GTP + 100 mM NaCl | 8,872 | 1,248 | 7 | 93% |

Several conclusions are reached from reviewing the results in Tables 1 and 2. First, the membrane bound complex between [125I]b-endorphin and its receptor is solubilized mostly in intact form. This is shown by the adsorption of a high proportion of the solubilized [125I]b-endorphin to immobilized WGA. Not only is a high proportion of the specifically bound radioligand adsorbed to WGA, as would be expected if it is bound to the receptor, but WGA selects for specifically bound material. This is shown by the large increase in the ratio of total cpm/nonspecific cpm in the WGA-bound material. Also, the soluble R:L complex is stable enough to be separated from free ligand in a step taking 2-3 hours. This predicts that a biotinylated b-endorphin is used to form a R:L complex that could be adsorbed in intact form to immobilized streptavidin.

Further, binding to immobilized WGA serves as an assay for the soluble R:L complex, and binding to WGA serves as a purification step for the receptor. Finally, the R:L complex is easily dissociated. This provides a means for eluting the receptor from an affinity column. For example, a soluble complex between the receptor and biotinyl b-endorphin could be bound to immobilized streptavidin and the receptor then eluted by GTP (partial elution), GTP + NaCl or NaCl.

The interactions of GTP with low concentrations of salt to cause dissociation of receptor and ligand is consistent with the known properties of opiate receptors. Later results in receptor purification experiments show that lower salt concentrations do not affect stability of this R:L complex and thus the GTP/salt interactions are synergistic.

Characterization of Biotinylated b-Endorphin

The two fractions of biotinylated b-endorphin are assayed for binding to rat brain opioid receptor by competition with $[^{125}I]$b-endorphin. The $IC_{50}$s for reduction of radioligand binding by competition with cold ligand are: b-endorphin, 1 nM; biotinyl-b-endorphin (F1), 1 nM; and biotinyl-b-endorphin (F2), 5 nM. Thus, both fractions of biotinylated b-endorphin show high affinity binding to opioid receptor. The F1 fraction consists of two peptides with molecular masses, identified by mass spectroscopy, of 3816 and 3875 daltons. The F1 fraction contains only the 3816 dalton species, the expected mass for biotinyl-b-endorphin. What is shown here is that heating the F1 fraction for 5 min. at 50°C eliminates the 3875 dalton species. Thus there is only one species of biotinyl-b- endorphin, with a mass of 3816 daltons, by mass spectrometry. Before further use, the F1 fraction is heated at 50°C for 5 min. This material has been reanalyzed for binding to receptors in rat brain membranes by competition vs. $[^{125}I]$b-endorphin and it binds with a protency very similar to that of b-endorphin. The IC508 are 1.2 nM for b-endorphin and 1.8 nM for biotinyl-b-endorphin.

Utility of Biotinylated b-Endorphin in Receptor Purification

Samples of brain membranes are incubated either with or without the F1 and F2 fractions of biotinyl-b-endorphin and carried through the procedure of solubilization, adsorption with immobilized streptavidin, elution and protein analysis by SDS-PAGE.

The WGA bound glycoprotein (WGA+) fractions of the eluates primarily contain a protein with MW about 66,000. Small amounts of this protein are seen in the material eluted by GTP-q-S and much larger amounts elute with the subsequent elution with 500 mM NaCl. The appearance of this band is ligand-dependent because it does not appear in eluates from the samples done without prior binding of biotinyl-b-endorphin. The nonglycosylated (WGA-) fractions show that GTP-q-S alone elutes nonglycosylated bands in the 30-40,000 MK (30-40K) range. These also occur only in samples incubated with biotinyl-b-endorphin and are thus ligand-dependent. Subsequent elution with 500 mM NaCl yields little if any further 30-40K MW material. Because both the F1 and F2 fractions give purification of the 66K band, they are used together in a 1:1 ratio in further experiments.

The elution of the 66K glycoprotein correlates with the effects of GTP-q-S on stability of the soluble R:L complex. Thus 100 mM GTP-q-S gives only partial dissociation of the soluble R:L complex and partial elution of the 66K glycoprotein. This band is considered to be the opioid receptor and will be referred to as such. It will also be referred to as "66K glycoprotein".

In a similar experiment, the 30-40K, GTP-q-S eluted protein specifically purified by biotinyl-b-endorphin is reactive with anti-G protein antiserum (Figure 4). In this experiment, biotinyl-NH-[Leu[8], D-Trp[22], Tyr[25]]-SRIF-28 (bio-S28); and biotinyl-b-endorphin are used to purify SRIF and opioid receptors, respectively by similar techniques. Both purifications employ essentially the same steps: binding of biotinylated ligand to intact membranes (from $GH_4C_1$ pituitary tumor cells and brain); solubilization of intact R:(bio)L complex; binding of R:(bio)L complex to streptavidin-immunoreactive material in the 40K size range only with the samples where receptor is complexed with biotinyl-ligand. Samples where the receptor is unoccupied or occupied by non-biotinyl ligand show no evidence of $G_{ia}$.

The ligand specificity of the 66K glycoprotein is further tested by blocking binding of the biotinylated b-endorphin with a large molar excess of nonbiotinylated ligand. When 100 nM biotinyl-b-endorphin (1:1 F1 + F2) is competed with a combination of 50 mM b-endorphin + 50 mM met-enkephalin, the yield of the 66K glycoprotein is greatly diminished. In another test of specificity, 40 mM naloxone effectively competes with 60 nM biotinyl-b-endorphin to nearly eliminate the recovery of 66K glycoprotein (Figure 5B). In this experiment, two additional bands are seen with MWs of about 140-160,000 and 50-55,000. Since both show ligand specificity they may be receptor subtypes, proteolytically degraded receptor or receptor aggregate.

12

While the 66K band is always the primary protein recovered by these methods, the relative amounts of the 140-160K and 50-55K bands are variable.

The SA-A column is first washed with 1 mM EDTA + 1 mM EGTA and then with 100 mM NaCl prior to elution with 500 mM NaCl. Since these wash steps carried out with very little loss of the 66K receptor band, they are incorporated into further procedures.

In the purifications shown above, elution of receptor from the SA-A columns is with 500 mM NaCl. This is used as an alternative to GTP because it elutes the 66K glycoprotein more effectively. However, the ability of the GTP-q-S + lower salt concentrations (25-100 mM NaCl) to dissociate the R:L complex suggests that it is possible to elute the receptor by avoiding high salt concentrations. This is tested by adsorbing the solubilized complex between receptor and biotinyl-b-endorphin to SA-A, dividing the sample into two different columns and eluting in two different ways as follows: A. elute sequentially with 100 mM GTP/75 mM NaCl and with 500 mM NaCl; B. wash with 100 mM NaCl and then elute with 500 mM NaCl. From the first SA-A column, elution with 100 mM GTP/75 mM NaCl yields nearly all of the 140-160K and 50-55K bands and a large proportion of the 66K band. The remainder of 66K material is eluted with 500 mM NaCl. From the second SA-A column, very little material is eluted by 100 mM NaCl while 500 mM NaCl elutes all of the ligand specific bands. Specificity is shown in this experiment by a naloxone block, where naloxone competes with biotinyl-b-endorphin for receptor binding and the 140-160K, 66K and 50-55K bands are not seen.

The results are significant for two reasons. First, they provide further correlations between the recovery of 66K protein and known properties of opioid receptors. A $NA^+$/GTP interaction is shown at two levels; by dissociation of the soluble R:L complex (Tables 1 and 2) and by recovery of specific receptor bands upon elution of affinity columns. Because the $NA^+$/GTP interaction is such a well documented property of opioid receptor binding, this data increases the probability that the 66K glycoprotein and other specific bands are, in fact, opioid receptor proteins. It is further shown that elution with 100 mM GTP/100 mM NaCl gives complete elution of the 66K band and other ligand specific species from the SA-A column. Thus subsequent elution with 500 mM NaCl yields little further ligand specific protein. However for routine receptor purification, elution with 500 mm Nacl alone provides a good yield of receptor which can be further purified on wheat germ agglutinin to yield sequencing quality receptor.

Pharmacology of Isolated Receptor

Further pharmaceutical analysis is done to determine the subtype of the 66 kDa receptor protein. Two different peptides, one known to exhibit mu-receptor selective binding ([D-Ala$^2$, N-MePhe$^4$, glyol$^5$]-enkephalin or DAGO; Bachem; 300 fold selectivity for mu over delta) and the other known to exhibit delta receptor selective binding ([D-Pen$^{2,5}$, pCL-Phe$^4$]enkephalin or pCl-DPDPE; 500-fold selectivity for delta over mu) are used to block binding of biotinyl-b-endorphin to rat brain membranes. This pair of ligands is appropriate because their affinities for their respective receptors are very similar (approximately 1 mM $K_D$). Each incubation contains 3 nM biotinyl-b-endorphin, and the blocking peptides are included at 50, 500, and 5000 nM. The ligand mixtures are incubated with unsolubilized membranes for one hour at room temperature and then purification of the receptor proceeds as described herein. A summary of the condition is provided in Table 3.

The ability of the respective peptides to block b-endorphin binding is determined by observing the relative recovery of biotinylated b-endorphin bound 66 kDa protein from each sample. It can be seen that the 66 kDa protein is recovered in about the same amounts from the control as when the pCl-DPDPE is used as a competitor. In contrast, DAGO blocked recovery of receptor almost completely at 500 nM and completely at 5000 nM, thereby confirming the identity of the protein as a mu-subtype opioid receptor.

EP 0 612 845 A2

## TABLE 3

| Sample | 3 nM Biotinyl-b-Endorphin | DAGO* | pCL- |
|---|---|---|---|
| DPDPE# | | | |
| 1 | + | - | - |
| 2 | + | 50 nM | - |
| 3 | + | 500 nM | - |
| 4 | + | 5000 nM | - |
| 5 | + | - | 50 nM |
| 6 | + | - | 500 nM |
| 7 | + | - | 5000 nM Notes: |

\* mu-specific
\# delta-specific

## Identification of 700 base pair C-DNA Clone pPCR4A

Analyses of products derived from PCR amplification of rat brain cDNA using oligonucleotides complementary to regions of the mouse =-opioid receptor identify the 700 base pair cDNA clone pPCR4A. This cDNA display 70% nucleotide sequence identity to the rat m-opioid receptor cDNA and homology with cDNA sequences of other G-linked receptor. One open reading frame of the pPCR4A sequence matches each of the 23 amino acids sequenced from a m-opioid receptor protein preparation.

Several rat cerebral cortical cDNAs hybridize with radiolabeled pPCR4A hybridization probes; one 2.2 kb cDNA termed RC8-1 is selected for further analysis. Sequence analyses reveals that 996 RC8-1 nucleotides encode an open reading frame of 332 amino acids with 63% amino acid identity to sequences of the m-opiate receptor, good homology to other neuropeptide receptors, and more distant homology to an "opiate binding protein" receptor, a catecholamine receptor and rhodopsin. Hydrophobicity analyses reveals at least seven hydrophobic putative membrane spanning domains of 20-24 amino acids whose sequences are especially conserved with other G-linked receptors. Threoninc residue 23 is found in a context especially favorable for protein kinase A phosphorylation. One consensus sequence for N-linked glycosylation at amino acid 11 is observed in the N-terminal domain (Z = 44).

COS cell expression of RC8-1 in the expression vector pcDNA1 yield naloxone-blockable, high affinity specific binding of [$^2$H] DAMGO and [$^3$H] DADLE binding saturation experiments are most consistent with a single population of binding sites for each ligand, with KK values of 0.4 and 0.5 nM, respectively. [$^2$H] DAMGO binding is reduced by addition of Na$^+$ or GTP to incubations, but not by adding ATP (Fig. 3). Mg$^{++}$ addition increases binding by COS cell expression of RC8-1 in the expression vector pcDNA1 yielded naloxone-blockable, high effinity specific binding of [$^3$H]DAMGO and [$^3$H]DADLE, with no appreciable specific recognition of [$^3$H]DPDPE or [$^3$H]U 69,593, that is not present in cells transfected with vector alone. Scatchard analysis of [$^3$H]DAMGO and [$^3$H]DADLE binding saturation experiments are most consistent with a single population of high affinity binding sites for each ligand, with Kn values on 0.4 and 0.5 nM respectively. [$^3$H]DAMGO binding is reduced by addition of Na+ of GTP to incubations. [$^3$H]DAMGO binding is displaced by a number of opioid compounds in stereoselective fashion. Pharmcologicallyactive (-) naloxone and dextrorphan isomers display substantially greater potency than pharmacologically less active (+) naloxone and dextrorphan isomers. Morphine, DADLE, (-) naloxone, naloxonazine, ethylketocyclazocino and bromazocino displace binding of [$^3$H]DAMGO with high potency (Table 1). DPDPE and p-C1-DEDPE, relatively 8-selective, U 50,488 and U 69,593, relatively k selective, display substantially less potency. These potencies display a good correlation with values described for affinities for the n-opioid receptor, but poor correlations with affinities documented at 8- or k- opioid receptors. Goldstein, A. &Naidu, A. (1989) Mol. Pharmacol. 36, 265-272. [$^3$H] DAMGO binding could be displaces by a number of opioid compounds in stereoselective fashion. Pharmacologically-active(-) naloxone and levorphan isomers display substantially greater potency than their pharmacologically-inactive isomers. Morphine displaces binding with high affinity shared by DADLe,(-) naloxone, naloxonazine, and bremazocine (Fig. 4A). DPDPE, relatively =-selective, and U-50,488, relatively =-selective, displayed substantially loss potencies. These protencies display a good correlation with values described for affinities for the m-opioid receptor, but poor correlations with affinities documented at =-opioid receptor.

14

Initial Northern analyses of the distribution of mRNA hybridizing with-radiolabeled RC8-1 hybridization probes suggests that relatively high expression levels of an 10.5 kb mRNA are found in the thalamus. RNase-protection assays of greater sensitivity are able to detect protected fragments consistent with significant mOR mRNA presence in the thalamus, cerebral cortex, straitum, hypothalamus, midbrain, hippocampus, brainstern, and spinal cord but not cerebellum or liver. In situ hybridization studies identify grain densities more than five-fold greater than autoradiographic background over cells in several thalamic nuclei that displayed the size, shape, and presence of nucleoli characteristic of neurons.

SEQUENCE LISTING

(1) GENERAL INFORMATION:

    (i) APPLICANT: Uhl Dr., George
                        Eppler Dr., Mark
                        Wang Dr., Jai-bei

    (ii) TITLE OF INVENTION: Purified Opioid Receptor
                                    & Nucleotide Sequence

    (iii) NUMBER OF SEQUENCES: 2

    (iv) CORRESPONDENCE ADDRESS:
        (A) ADDRESSEE: American Cyanamid Company
        (B) STREET: 1937 West Main Street
        (C) CITY: Stamford
        (D) STATE: CT
        (E) COUNTRY: United States of America
        (F) ZIP: 06904-0060

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER: US
        (B) FILING DATE: 11-JUNE-1993
        (C) CLASSIFICATION:

    (vii) PRIOR APPLICATION DATA:
        (A) APPLICATION NUMBER: US 07/855,286
        (B) FILING DATE: 23-MAR-1992

    (viii) ATTORNEY/AGENT INFORMATION:
        (A) NAME: Tsevdos Dr., Estelle J.
        (B) REGISTRATION NUMBER: 31,145
        (C). REFERENCE/DOCKET NUMBER: 31849-02

    (ix) TELECOMMUNICATION INFORMATION:
        (A) TELEPHONE: 203-321-2756
        (B) TELEFAX: 203-321-2971
        (C) TELEX: 710-474-4059

(2) INFORMATION FOR SEQ ID NO:1:

    (i) SEQUENCE CHARACTERISTICS:
        (A) LENGTH: 23 amino acids.
        (B) TYPE: amino acid
        (C) STRANDEDNESS: single
        (D) TOPOLOGY: linear

    (ii) MOLECULE TYPE: peptide

16

```
(iii) HYPOTHETICAL: NO

(iv) ANTI-SENSE: NO


(xi) SEQUENCE DESCRIPTION: SEQ ID NO:1:

    Lys Glu Lys Asp Arg Asn Leu Arg Arg Ile Thr Arg Met Val Leu
Val
    1               5                   10                  15


    Val Val Ala Val Phe Ile Val
                20

(2) INFORMATION FOR SEQ ID NO:2:

    (i) SEQUENCE CHARACTERISTICS:
        (A) LENGTH: 31 amino acids
        (B) TYPE: amino acid
        (C) STRANDEDNESS: single
        (D) TOPOLOGY: linear

    (ii) MOLECULE TYPE: peptide

    (iii) HYPOTHETICAL: NO

    (iv) ANTI-SENSE: NO


    (xi) SEQUENCE DESCRIPTION: SEQ ID NO:2:

    Tyr Gly Gly Phe Met Thr Ser Glu Lys Ser Gln Thr Pro Leu Val
Thr
    1               5                   10                  15


    Leu Phe Lys Asn Ala Ile Ile Lys Asn Ala Tyr Lys Lys Gly Glu
                20                  25                  30
```

## Claims

1. An isolated purified nucleic acid sequence encoding a mu subtype opioid receptor or biologically active fragments thereof.

2. The sequence of Claim 1, which comprises the sequence depicted in Figure 11, or which hybridizes with a nucleic acid sequence encoding the amino acid sequence depicted in figure 9 or biologically active fragments thereof under standard hybridization conditions.

3. The sequence of claim 1 which encodes the amino acid sequence of Figure 9 or biologically active fragments thereof.

4. An expression vector comprising the isolated nucleic acid sequence of Claim 1.

5. The vector of Claim 4 which is a plasmid.

6. A host cell transformed with the nucleic acid sequence of Claim 1.

7. A host cell comprising the vector of claim 4.

8. A method of recombinantly preparing a substantially pure opioid receptor which comprises the steps of
(a) introducing a recombinant vector comprising the DNA sequence of claim 2 into a suitable host cell,
(b) culturing the resulting transformed host cell in a suitable culturing medium, and
(c) isolating the recombinant mu-subtype opioid receptor from the cultured cells.

9. The method of claim 8 in which the opioid receptor is a mu-subtype opioid receptor.

10. A substantially pure mu-subtype opioid receptor, or a biologically active fragment thereof.

11. The receptor of claim 10 which binds with $\beta$-endorphin.

12. The receptor of Claim 11 which has a molecular weight of about 66,000 daltons.

13. The receptor of Claim 10 which comprises the amino acid sequence Lys-Gly-Lys-Asp-Arg-Asn-Leu-Arg-Arg-Ile-Thr-Arg-Met-Val-Leu-Val-Val-Val-Ala-Val-Phe-Ile-Val.

14. A substantially pure mu-subtype opioid receptor which is isolatable by:
a. contacting membranes of cells which express a mu-subtype opioid receptor with a biotinylated ligand which binds the opioid receptor to form a receptor:ligand complex;
(b) solubilizing the membranes containing receptor:ligan complex;
(c) contacting the solubilized receptor:ligand complex with a substrate capable of binding the biotinylated ligand;
(d) contacting the substrate-bound receptor:ligand complex with an eluant capable of releasing the receptor from the receptor:ligand complex;
(e) contacting the eluted receptor with a lectin affinity substrate;
(f) recovering a purified receptor by elution from the lectin affinity substrate.

15. The receptor of Claim 14 wherein the ligand is biotinylated-$\beta$-endorphin.

16. The receptor of Claim 15 in which the eluant of step (d) contains a GTP analog.

17. The receptor of Claim 16 in which the eluant also contains NaC1.

18. The receptor of Claim 16 in which the eluant contains about 100 $\mu$M GTP-$\lambda$-S and about 100 mM NaC1.

19. An antibody which reacts specifically with a mu-subtype opioid receptor.

FIGURE 1

Receptor Site

H₂N-Tyr-Gly-Gly-Phe-Met-Thr-Ser-Glu-Lys-Ser-Gln-Thr-Pro-Leu-Val-Thr-Leu-
Phe-Lys-Asn-Ala-Ile-Ile-Lys-Asn-Ala-Tyr-Lys-Lys-Gly-Glu-[biotinyl-Lys³²]

Streptavidin Site

FIGURE 2

EP 0 612 845 A2

FIGURE 3

EP 0 612 845 A2

FIGURE 4

22

A.

```
              +    +     10⁻⁷ M bio-β-endo.
              -    +     10⁻⁴ M β-endo./met-enk.
    MW
```

```
   66-
```

B.

```
              +    +    6×10⁻⁸ M bio-β-endo
    MW        -    +    4×10⁻⁵ M Naloxone
```

```
   205 -
   98  -
   66  -


   45  -
```

FIGURE 5

FIGURE 6

FIGURE 7

Figure 8

FIGURE 9

Revised 6/8/93

```
        1                                                       50
                                                                 ⌇
Muorl   .......... .......... .......... .........M RSEPTGLGGN
DOR-1   .......... ......MEL VPSARAELQS SPLVNLSDAF PSAFPSAGAN
SOMAT   .......... .....MFPNA PPPLPHSSPS SSPGGCGEGV CSRGPGSGAA
F-PEP   .......... .......... .......... .......... METNSSLPTN
OPB-R   .......... ......MASP AGNLS.AWPG WGWPP..PAA LRNLTSSPAP
NEU-K   MASVPRGENW TDGTVEVGTH TGNLSSALGV TEWLALQAGN FSSALGLPAT
RHODOP  .......... .....MNGTE GPNFYVPFSN .......... ........AT
B2-ADR  .......... .......... .......... .......... ......MEPH


        51                                                     100
Muorl   DSLCPQTGS. ......P.... ....SMVTGI TIMALYSIVC VVGLFGNFLV
DOR-1   ASGSPGARS. ......AS... ....SLALAI AITALYSAVC AVGLLGNVLV
SOMAT   DGMEEPGRN. .....SSQNG TLSEGQGSAI LISFIYSVVC LVGLCGNSMV
F-PEP   ISGGTPAVS. .....AGY.. .....LFLDI ITYLVFAVTF VLGVLGNGLV
OPB-R   TASPSPAPSW TPSPRPGPAH PPLQPPWAVA LWSLAYGAVV AVAVLGNLVV
NEU-K   TQAPSQV... ....RANLTN QFVQPSWRIA LWSLAYGLVV AVAVFGNLIV
RHODOP  GVVRSPF... ......EYPQ YYLAEPWQFS MLAAYMPLLI VLGFPINFLT
B2-ADR  GNDSDFLLAP NGSRAPGHDI TQERDEAWVV GMAILMSVIV LAIVFGNVLV


        101                                                    150
Muorl   MYVIVRYTKM KTATNIYIFN LALADALATS TLP.FQSVNY LMGTWPFGTI
DOR-1   MFGIVRYTKL KTATNIYIFN LALADALATS TLP.FQSAKY LMETWPFGEL
SOMAT   IYVILRYAKM KTATNIYILN LAIADELLML SVP.FLVTST LLRHWPFGAL
F-PEP   IWVAG.FRMT HTVTTISYLN LAVADFCFTS TLPFFMVRKA MGGHWPFGWF
OPB-R   IWIVLAHKRM RTVTNSFLVN LAFADAAMAA LNALVNFIYA LHGSWYFGAN
NEU-K   IWIILAHKRM RTVTNYFLVN LAFSDASVAA FNTLINFIYG LHSEWYFGAN
RHODOP  LYVTVQHKKL RTPLNYILLN LAVADLFMVF GGFTTTLYTS LHGYFVFGPT
B2-ADR  ITAIAKFERL QTVTNYFITS LACADLVHGL AVVPFGASHI LMKMWNFGNF


        151                                                    200
Muorl   LCKIVISIDY YNMFTSIFTL CTMSVDRYIA VCHPVKALDF RTPRNAKIVN
DOR-1   LCKAVLSIDY YNMFTSIFTL TMMSVDRYIA VCHPVKALDF RTPAKAKLIN
SOMAT   LCRLVSVDA VNMFTSIYCL TVLSVDRYVA VEHPIKAARY RRPTVAKVVN
F-PEP   LCKFVFTIVD INLFGSVFLI ALIALDRCVC VLHPVWTQNH RTVSLAKKVI
OPB-R   YCRFQNFFPI TAVFASIYSM TAIAVDRYMA IIDPLKP.RL .SATATRIVI
NEU-K   YCRFQNFFPI TAVFASIYSM TAIAVDRYMA IIDPLKP.RL .SATATKIVI
RHODOP  GCNLEGFFAT LGGEIALWSL VVLAIERYVV ICKPMSNFRF .GENHAIMGV
B2-ADR  WCEFWTSIDV LCVTASIETL CVIAVDRYVA ITSPFKYQSL LTKNKARVVI


        201                                                    250
Muorl   VCNWILSSSA IGLPVMVMAV TKY.R QGSIDCTLTF SHPTWYWENL
DOR-1   ICIWVLA.SG VGVPIMV... ..MAVTQP.R DGAVVCMLQF PSPSWYWDTV
SOMAT   LGVWVLS.LL VILPIVV... ..FSRTAANS DGTVACNMLM PEPAQRWLVG
F-PEP   IGPWVMA.LL LTLPVII... ..RVTTVPGK TGTVACTFNF SPWTNDPKER
OPB-R   GSIWILA.FL LAFPQCL......YSKIKVM PGRTLCYVQW PEGSRQHFTY
NEU-K   GSIWILA.FL LAFPQCL......YSKIKVM PGRTLCYVQW PEGPKQHFTY
RHODOP  VFTWIMA.LA CAAPPLVGWS RYIPEGMQCS CGVD.YYTLK PEVNNESFVI
B2-ADR  LMVWIVSGLT SFLPIQMHWY RATHRQAIDC YAKETCCDPF TNQAY.....


        251                                                    300
Muorl   LKICVFIFAF VVPILIITVC YGLMILRLK SVRMLSGSKE
DOR-1   TKICV..... .....FLFAF VVPILIITVC YG.LMLLRLR SVRLLSGSKE
SOMAT   FVLYT..... .....FLMGF LLPVGAICLC YV.LIIAKMR MVPSRPAG.S
F-PEP   IKVAVAMLTV RGIIRFIIGF SAPMSIVAVS YG.LIATKIH KQGLIKSS..
OPB-R   HMIVI..... ....VLVYCF ..PLLIHGIT YT.IVGITLW GGEIPGDTCD
NEU-K   HIIVI..... ....ILVYCF ..PLLIHGVT YT.IVGITLW GGEIPGDTCD
RHODOP  YMFVV..... ........HF TIPLIVIFFC YG.QLVFTVK EAAAQQQESA
B2-ADR  .......... .AIASSIVSF YVPLVVMVFV YSRVFQVAKR QLQKIDKSEG
```

27

CONTINUATION OF FIGURE 9

```
      301                                        *              350
Muorl  K....DRN..  ..........  ..........L RRITRMVLVV VGAFVVCWAP
DOR-1  K....DRS..  ..........  ..........L RRITRMVLVV VGAFVVCWAP
SOMAT  T....QRS..  ..........  ..........E RKITLMVMMV VMVFVICWMP
F-PEP  ..........  ..........  ..........  .RPLRVLSFV AAAFFLCWSP
OPB-R  KYQEQLKA..  ..........  ..........K RKVVKMMIIV VVTFAICWLP
NEU-K  KYHEQLKA..  ..........  ..........K RKVVKMMIIV VVTFAICWLP
RHODOP TTQ...KA..  ..........  ..........E KEVTRMVILM VVFFLICWFP
B2-ADR RFHAQNLSQV EQDGRSGHGL RSSSKFCLKE HKALKTLGII MGTFTLCWLP

      351                                                      400
Muorl  IHIFVIVWTL VDINRRDFL. ..QTVSWH.. .......... .NPVLYAFLDE
DOR-1  IHIFVIVWTL VDINRRDFL. ..VVAALHLC TALGYANSSL NPVLYAFLDE
SOMAT  FYVVQLVNVF AEQDDATV.. ......SQLS VILGYANSCA NPILYGFLSD
F-PEP  YQVVALIATV RIRELLQGMY KEIGIAVDVT SALAFFNSCL NPMLYVFMGQ
OPB-R  YH..IYFILT AIYQQLNRWK YIQQVYLA.S FWLAHSSTMY NPIIYCCLNK
NEU-K  YH..VYFILT AIYQQLNRWK YIQQVYLA.S FWLAHSSTMY NPIIYCCLNK
RHODOP YAGVAFYIFT ..HQGSN... .FGPIFMTLP AFFAKSSSIY NPVIYIMMNK
B2-ADR FFIVNIVHVI RA.......N LIPKEVYILL NWLGYVNSAF NPLIYC.RSP

      401                                                      450
Muorl  NFKRCF.REF CIPTSSTIE. .QQNSTRVRQ NTREHPSTAN TVDRTNHQLE
DOR-1  NFKRCF.RQL CRTPCGRQE. .PGSLRRPRQ ATTRERVTAC TPS......D
SOMAT  NFKRSFQRIL CL...SWMD. .NAAEEPVDY YATALKSRAY SVE..DFQPE
F-PEP  DFRERLIHAL PASLERALT. .EDSTQTSDT ATNSTLPSAE VALQAK....
OPB-R  RFRAGFKRAF RWCPFIHVSS YDELELKATR LHPMRQSSLY TVTRMESMSV
NEU-K  RFRAGFKRAF RWCPFIQVSS YDELELKTTR FHPTRQSSLY TVSRMESVTV
RHODOP QFRN...... .......... .......... .........C MLTTLCCGKN
B2-ADR DFRIAFQELL CLRRSSSKTY GNGYSSNSNG RTDYTGEQSA YQLGQEKENE

      451                                                      500
Muorl  NLEAETAPLP .......... .......... .......... ..........
DOR-1  GPGGGRAA.. .......... .......... .......... ..........
SOMAT  NLESGGVFRN GTCASRISTL .......... .......... ..........
F-PEP  .......... .......... .......... .......... ..........
OPB-R  VFDCNDGDSA RSSHQKRGTT RDVGSNVCSR RNSKSTSTTA SFVSSSHMSV
NEU-K  LFDPNDGDPT KSSRKKRAVP RDPSANGCSH RGSKSASTTS SFISSPYTSV
RHODOP ILGDDEASAT ASKTETSQVA PA........ .......... ..........
B2-ADR LLCEEAPGME GFVNCQGTVP SLSIDSQGRN CNTNDSPL.. ..........

      501
Muorl  ....
DOR-1  ....
SOMAT  ....
F-PEP  ....
OPB-R  EEGS
NEU-K  DEYS
RHODOP ....
B2-ADR ....
```

28

FIGURE 10

FIGURE 11


Linear) MAP of: Rc8-1.Fin   check: 679   from: 1  to: 2070

REFORMAT of: Rc8-1.Fin   check: -1   from: 1  to: 2072   June 2, 1993   09:59
(No documentation)

With 186 enzymes: *

```
                        June 7, 1993   12:27  ..

                     B   B                        E     E
        ET           p   pC                       cA PSCNc S   C
        Acs          uD  AuvD                     olBfcvlo c   v
        pop          1d  11id                     RwalriaR r   i
        oRE          0e  u0Je                     IN1MFJII F   J
        III          II  IIII                     IIIIIIVI I   I
         /            /   //                          ///
        GAATTCCCAGACCCCCTTAGCTCAGGCAAGTTGCTCCCCAGCACCTGGCTCCTGGCTCAAC
    1   ---~-----+---------+---------+---------+---------+---------+  60
        CTTAAGGGTCTGGGGAATCGAGTCCGTTCAACGAGGGGTCGTGGACCGAGGACCGAGTTG

b       N   S   Q   T   P   *   L   R   Q   V   A   P   Q   H   L   A   P   G   S   T   -

                                         S         N
            M       B                     a         l                   B   CS
            a   M s B           BA        u D       Aa                  sMFNvc   A
            e   s t c           sl        3 p       cI                  isacir   c
            I   1 X c           rw        A n       iI                  EpuiJF   i
            I   I I I           II        I I       II                  IIIIII   I
                                             /                          / / /
        TTGTCCACGTTGATGGCAACCAGTCCGATCCATGCGGTCTGAACCGACCGGGCTTGGCGG
   61   --------+---------+---------+---------+---------+---------+  120
        AACAGGTGCAACTACCGTTGGTCAGGCTAGGTACGCCAGACTTGGCTGGCCCGAACCGCC

b       C   P   R   *   W   Q   P   V   R   S   M   R   S   E   P   T   G   L   G   G   -

                        B
                        s
                        p                 F                 `M N T
            C           1       B     n C       B     a l s     C
            v           2D      BsM  Mu v       sBDBeNaSp       v           BX
            i           8d      srs  n4 i       assbIcIt4       i           cc
            J           6e      1Fp  1H J       JlavIoIy5       J           cm
            I           II      III  II I       IIIIIIIII       I           II
                                 //                     /  ////              /
        GAACGACAGCCTGTGCCCTCAGACCGGCAGCCCTTCCATGGTCACAGCCATTACCATCAT
  121   --------+---------+---------+---------+---------+---------+  180
        CTTGCTGTCGGACACGGGAGTCTGGCCGTCGGGAAGGTACCAGTGTCGGTAATGGTAGTA

b       N   D   S   L   C   P   Q   T   G   S   P   S   M   V   T   A   I   T   I   M   -

        N HS                             S   H                   E               N
        1Caa                         M   a   Ca                  c   S           l
        aveu            M            b   u   ve      E   M       o   c           aM
        IiI9            n            o   9   iI      a   n       R   r           Is
        IJI6            l            I   6   JI      r   l       I   F           Il
        IIII            I            I   I   II      I   I       I   I           II
         //                                           /
        GGCCCTCTACTCTATCGTCTGTGTAGTGGGCCTCTTCGGAAACTTCCTGGTCATGTATGT
  181   --------+---------+---------+---------+---------+---------+  240
        CCGGGAGATGAGATAGCACACACATCACCCGGAGAAGCCTTTGAAGGACCAGTACATACA
```

FIGURE 11

```
b       A  L  Y  S  I  V  C  V  V  G  L  F  G  N  F  L  V  M  Y  V  -

                                              M
                     M              B         b                    B
                     s              b         o                    s
                     l              s         I                    l
                     I              I         I                    I
        GATTGTAAGATACACCAAAATGAAGACTGCCACCAACATCTACATTTTCAACCTTGCTCT
    241 ---------+---------+---------+---------+---------+---------+ 300
        CTAACATTCTATGTGGTTTTACTTCTGACGGTGGTTGTAGATGTAAAAGTTGGAACGAGA


b       I  V  R  Y  T  K  M  K  T  A  T  N  I  Y  I  F  N  L  A  L  -

                  B
              B   p                                    H
              s   uD  H  B  R                          i
              a   1d  g  s  s                          n        B
              H   0e  a  r  a                          c        c
              I   II  I  I  I                          I        I
                    /
        GGCAGACGCCTTAGCGACCAGTACACTGCCCTTTCAGAGTGTCAACTACCTGATGGGAAC
    301 ---------+---------+---------+---------+---------+---------+ 360
        CCGTCTGCGGAATCGCTGGTCATGTGACGGGAAAGTCTCACAGTTGATGGACTACCCTTG


b       A  D  A  L  A  T  S  T  L  P  F  Q  S  V  N  Y  L  M  G  T  -

        N HS                    S     S                      A  N
         1Caa       ND          C  a  Ba  D                  f  l
         Faveu      1r  B       v  u  DM su  D               H  aN
         oIiI9      ad  c       i  3  pn a3  p               p  Is
         kIJI6      II  c       R  A  nl BA  n               h  Ip
         IIIII      VI  I       I  I  II II  I               I  II
          //                          /                            /
        ATGGCCCTTCGGAACCATCCTCTGCAAGATCGTGATCTCAATAGATTACTACAACATGTT
    361 ---------+---------+---------+---------+---------+---------+ 420
        TACCGGGAAGCCTTGGTAGGAGACGTTCTAGCACTAGAGTTATCTAATGATGTTGTACAA


b       W  P  F  G  T  I  L  C  K  I  V  I  S  I  D  Y  Y  N  M  F  -

                              N     S
                        C     Bl B  AaB
              BH        v     MsaM s vusA                          B
              sp        i     npIs t a9pc                          s
              gh        R     1WI1 X I6Wi                          r
              II        I     IIII I IIII                          I
               /                    /
        CACCAGCATATTCACCCTCTGCACCATGAGCGTGGACCGCTACATTGCTGTCTGCCACCC
    421 ---------+---------+---------+---------+---------+---------+ 480
        GTGGTCGTATAAGTGGGAGACGTGGTACTCGCACCTGGCGATGTAACGACAGACGGTGGG


b       T  S  I  F  T  L  C  T  M  S  V  D  R  Y  I  A  V  C  H  P  -

                   T
                E  a
                CBcSq                          H
                vsocI          R               i  M     C     B
                iaRrI          s               n  a     vA    a
                JJIF-          a               c  e     il    m
                IIII2          I               I  I     Rw    H
                                               I  I     II    I
```

31

FIGURE 11

```
              /
     AGTCAAAGCCCTGGATTTCCGTACCCCCCGAAATGCCAAAATCGTCAACGTCTGCAACTG
481  ---------+---------+---------+---------+---------+---------+ 540
     TCAGTTTCGGGACCTAAAGGCATGGGGGGCTTTACGGTTTTAGCAGTTGCAGACGTTGAC

b      V   K   A   L   D   F   R   T   P   R   N   A   K   I   V   N   V   C   N   W   -
                   T
               S   a
           B   MNa q
           sBDblu AI    EM  B                           N
           tspoa3 lI    an  c                           l
           YrnIIA w-    rl  c                           a
           IIIIVI I1    II  I                           I
           / ////  /                                    I
                                                        I
     GATCCTCTCTTCTGCCATCGGTCTGCCTGTAATGTTCATGGCAACCACAAAATACAGGCA
541  ---------+---------+---------+---------+---------+---------+ 600
     CTAGGAGAGAAGACGGTAGCCAGACGGACATTACAAGTACCGTTGGTGTTTTATGTCCGT

b      I   L   S   S   A   I   G   L   P   V   M   F   M   A   T   T   K   Y   R   Q   -
                                                                     T
               S                                        EH          a
           ANa             C       M                     cgS         q
           vlu             v       a       M             oic  X  RB  I  B
           aa9             i       e       n             REr  c  ss  I  s
           II6             R       I       l             IIP  m  al  -  r
           IVI             I       I       I             III  I  II  2  I
               /                                         /
     GGGGTCCATAGATTGCACCCTCACGTTCTCCCACCCAACCTGGTACTGGGAGAACCTGCT
601  ---------+---------+---------+---------+---------+---------+ 660
     CCCCAGGTATCTAACGTGGGAGTGCAAGAGGGTGGGTTGGACCATGACCCTCTTGGACGA

b      G   S   I   D   C   T   L   T   F   S   H   P   T   W   Y   W   E   N   L   L   -
                                           N  S                   D   M
           B           M                   B  1Aa                 r   a
           s           b                   sMavu                MM a   e
           p           o                   rsIa9                ns I   I
           M           I                   FpII6                11 I   I
           I           I                   IIIII                II I   I
                                              / /                   / /
     CAAAATCTGTGTCTTTATCTTCGCTTTCATCATGCCGGTCCTCATCATCACTGTGTGTTA
661  ---------+---------+---------+---------+---------+---------+ 720
     GTTTTAGACACAGAAATAGAAGCGAAAGTAGTACGGCCAGGAGTAGTAGTGACACACAAT

b      K   I   C   V   F   I   F   A   F   I   M   P   V   L   I   I   T   V   C   Y   -
                                                                         B
                                                                         a
           HB      S                                   N                 p
           Cac     a           B  H                    C  1              CNB1
           vee     u  DP        c  i           M        a  aNS            vla2
           iI8     3  pl        e  n           l        c  Isp            ian8
           JI3     A  ne        f  f           y        8  Iph            JII6
           III     I  II        I  I           I        I  III            IVII
              /                                            //                /
     CGGCCTGATGATCTTACGACTCAAGAGCGTTCGCATGCTATCGGGCTCCAAAGAAAAGGA
721  ---------+---------+---------+---------+---------+---------+ 780
     GCCGGACTACTAGAATGCTGAGTTCTCGCAAGCGTACGATAGCCCGAGGTTTCTTTTCCT
```

FIGURE 11

```
b        G   L   M   I   L   R   L   K   S   V   R   M   L   S   G   S   K   E   K   D   -

                             S
             H               a
             iT      HFH     u  D      MAMNcB          F           C           F
             nf      psh     3  p      slscrc          o           v           o
             fi      hpa     A  n      pwliFc          k           i           k
             II      III     I  I      IIIIII          I           J           I
                                                                   I
                  /                         ///
        CAGGAATCTGCGCAGGATCACCCGGATGGTGCTGGTGGTCGTGGCTGTATTTATCGTCTG
   781  ---------+---------+---------+---------+---------+---------+ 840
        GTCCTTAGACGCGTCCTAGTGGGCCTACCACGACCACCAGCACCGACATAAATAGCAGAC


b        R   N   L   R   R   I   T   R   M   V   L   V   V   V   A   V   F   I   V   C   -

                                                       E
                                                       c
                 S                                     o   S
             ABNa                    M                 4 H a       H
             vslu       B            a                 7HaBuD      iT
             apa9       c            e                 Ihec3p      nf
             IGI6       c            I                 IaIlAn      fi
             IIVI       I            I                 IIIIII      II
             /  /                                          /           /
        CTGGACCCCCATCCACATCTACGTCATCATCAAAGCGCTGATCACGATTCCAGAAACCAC
   841  ---------+---------+---------+---------+---------+---------+ 900
        GACCTGGGGGTAGGTGTAGATGCAGTAGTAGTTTCGCGACTAGTGCTAAGGTCTTTGGTG


b        W   T   P   I   H   I   Y   V   I   I   K   A   L   I   T   I   P   E   T   T   -

                     E                                 M               FN
                     c S                    C          a               CnsP      H
                     oBc                    v          Be              Avupv     iT
                     Rsr                    i          bI              li4Bu     nf
                     IlF                    R          vI              uJHII     fi
                     III                    I          II              IIIII     II
                                                          /               ////     /
        ATTTCAGACCGTTTCCTGGCACTTCTGCATTGCTTTGGGTTACACGAACAGCTGCCTGAA
   901  ---------+---------+---------+---------+---------+---------+ 960
        TAAAGTCTGGCAAAGGACCGTGAAGACGTAACGAAACCCAATGTGCTTGTCGACGGACTT


b        F   Q   T   V   S   W   H   F   C   I   A   L   G   Y   T   N   S   C   L   N   -

                             E           E
                             c S         cS                                    C
             B               o c         of          F           F             v
             s               R r         5a          o           o             i
             r               I F         7N          k           k             R
             I               I I         II          I           I             I
        TCCAGTTCTTTACGCCTTCCTGGATGAAAACTTCAAGCGATGCTTCAGAGAGTTCTGCAT
   961  ---------+---------+---------+---------+---------+---------+ 1020
        AGGTCAAGAAATGCGGAAGGACCTACTTTTGAAGTTCGCTACGAAGTCTCTCAAGACGTA


b        P   V   L   Y   A   F   L   D   E   N   F   K   R   C   F   R   E   F   C   I   -

                             S
             S               a  B                          H
             f               uMDsPT            M  ATXi           PF        B
             a               3npiva            l  vahn           lo        f
```

33

FIGURE 11

```
        N                AlnEuq              y   aqof         ●k        ▲
        I                IIIIII              I   IIII         II        I
                           //                    /
        CCCAACCTCGTCCACGATCGAACAGCAAAACTCCACTCGAGTCCGTCAGAACACTAGGGA
1021    ---------+---------+---------+---------+---------+---------+ 1080
        GGGTTGGAGCAGGTGCTAGCTTGTCGTTTTGAGGTGAGCTCAGGCAGTCTTGTGATCCCT

b       P  T  S  S  T  I  E  Q  Q  N  S  T  R  V  R  Q  N  T  R  E  -

                              S
            B   C             a  B                  C
            sD   v M          u  cDT     A          ABv     M
            as   i n          3  epa     l          lfi     n
            Ja   J l          A  fnq     w          uaJ     l
            II   I I          I  III     I          III     I
             /                    //                 /
        ACATCCCTCCACGGCTAATACAGTGGATCGAACTAACCACCAGCTAGAAAATCTGGAGGC
1081    ---------+---------+---------+---------+---------+---------+ 1140
        TGTAGGGAGGTGCCGATTATGTCACCTAGCTTGATTGGTGGTCGATCTTTTAGACCTCCG

b       H  P  S  T  A  N  T  V  D  R  T  N  H  Q  L  E  N  L  E  A  -

                                                            B
                                                            p  H
                                                            u  i
            AAB                         B                    l  n  C
            lps         B      FB   B   Bs   B                1DdMAvDM
            wap         p      os   s   sm   c                0dInlidn
            NBW         m      kl   r   aA   c                2eIluJel
            III         I      II   I   II   I                IIIIIII
             /                          /                     / /  ///
        AGAAACTGCTCCATTGCCCTAACTGGGTCTCACACCATCCAGACCCTCGCTAAGCTTAGA
1141    ---------+---------+---------+---------+---------+---------+ 1200
        TCTTTGACGAGGTAACGGGATTGACCCAGAGTGTGGTAGGTCTGGGAGCGATTCGAATCT

b       E  T  A  P  L  P  *  L  G  L  T  P  S  R  P  S  L  S  L  E  -

            FH
        C na         MBB   H              R                C
        vAue  B      Mass  iT             l         M      v           D
        ic4I  c      seat  nf             e         n      i           d
        JiHI  c      lIAX  fi             A         l      J           e
        IIII  I      IIII  II             I         I      I           I
         //           /    /
        GGCCGCCATCTACGTGGAATCAGGTTGCTGTCAGGGTGTGTGGGAGGCTCTGGTTTCCTG
1201    ---------+---------+---------+---------+---------+---------+ 1260
        CCGGCGGTAGATGCACCTTAGTCCAACGACAGTCCCACACACCCTCCGAGACCAAAGGAC

b       A  A  I  Y  V  E  S  G  C  C  Q  G  V  W  E  A  L  V  S  *  -

                S                                                    N
                a          C                                   CS    l
            AB  u  D      Bv                   B     v  M      vf    a
            lc  3  p      si                   s     i  n      ia    I
            wc  A  n      mR                   g     J  l      RN    I
            II  I  I      II                   I     I  I      II    I
                           /                                   /
        AGAAACCATCTGATCCTGCATTCAAAGTCATTCCTCTCTGGCTACTTCACTCTGCACATG
1261    ---------+---------+---------+---------+---------+---------+ 1320
        TCTTTGGTAGACTAGGACGTAAGTTTCAGTAAGGAGAGACCGATGAAGTGAGACGTGTAC
```

FIGURE 11

```
b     E   T   I   *   S   C   I   Q   S   H   S   S   L   A   T   S   L   C   T   *   -

                                          B               B   M               H
                      D                RSD  E   a         sM  b              iT
                      d                scd  a   m         as  o              nf
                      e                aae  r   A         Wp  I              fi
                      I                III  I   I         II  I              II
                                            /                                /
      AGAGATGCTCAGACTGTATCAAGTACTCAGAAAGAAGAGACTACCGGACACTCCTGAATC
1321  --------+---------+---------+---------+---------+---------+ 1380
      TCTCTACGAGTCTGACATAGTTCATGAGTCTTTCTTCTCTGATGGCCTGTGAGGACTTAG


b     E   M   L   R   L   Y   Q   V   L   R   K   K   R   L   P   D   T   P   E   S   -

              B
              s
              pN                            D   S           T
          C   11          D                 r   Aa          a                   A
          Av  4aR         r   B         B   a   vu          q                   p
          li  0Is         d   c         s   I   a9          I                   o
          uJ  7Ia         I   c         r   I   I6          I                   I
          II  III         I I           I I  II            2                   I
          /                                      /
      CAGCTCATGTACAGAACCATCTGAAACACCCAGTGGACCACAATGCTCTGTGGTATGTGA
1381  --------+---------+---------+---------+---------+---------+ 1440
      GTCGAGTACATGTCTTGGTAGACTTTGTGGGTCACCTGGTGTTACGAGACACCATACACT


b     S   S   C   T   E   P   S   E   T   P   S   G   P   Q   C   S   V   V   C   E   -

              S               BMT
      T       a               sas                       T
      s       Tu  D           tep         H       M   A  s
      p       a3  p           EI4         p       n   p  p
      E       qA  n           II5         h       l   o  E
      I       II  I           III         I       I   I  I
                              /
      ATTTCGATCATCATAGAAGGTGACCCCTCTCTATGTAGAATTTTTATTTTTCAAGCAAAT
1441  --------+---------+---------+---------+---------+---------+ 1500
      TAAAGCTAGTAGTATCTTCCACTGGGGAGAGATACATCTTAAAAATAAAAAGTTCGTTTA


b     F   R   S   S   *   K   V   T   P   L   Y   V   E   F   L   F   F   K   Q   I   -

              T
              t
              h
              l                   M   T
              l                   a   s
              l           M       e   p           M   A  s       S   M
              I           n       I   4           s   p  p       f   s
              I           l       I   5           e   o  E       c   l
              I           I       I   I           I   I  I       I   I
      ACTTATGACCTCATCAAAGAAAATAATGTCACTTGTTAAATTCACTGTAGTGATACATAA
1501  --------+---------+---------+---------+---------+---------+ 1560
      TGAATACTGGAGTAGTTTCTTTTATTACAGTGAACAATTTAAGTGACATCACTATGTATT


b     L   M   T   S   S   K   K   I   M   S   L   V   K   F   T   V   V   I   H   K   -

                                      T
                                      tM  T
                                      ha  s
```

FIGURE 11

```
                        MH      BleMp        S              B
                        np      slIn4        f              s
                        lh      rlIl5        c              r
                        II      IIIII        I              I
                                  / /
        AGTAAATGCTACCTCTGACCTCTGACCCAGTCACCTTCTGTAGAGAGTTCCAGTCCTTTT
1561    ---------+---------+---------+---------+---------+---------+  1620
        TCATTTACGATGGAGACTGGAGACTGGGTCAGTGGAAGACATCTCTCAAGGTCAGGAAAA

b        V   N   A   T   S   D   L   *   P   S   H   L   L   *   R   V   P   V   L   L   -

             B                       MH                  M           B   M
             c                       sp                  m           f   s
             c                       eh                  e           a   e
             I                       II                  I           I   I
        GTGATGGAATACATCATTTCCAACTTAAAACTTTCACCTTGAAGTTATGGTCTAGTTAAG
1621    ---------+---------+---------+---------+---------+---------+  1680
        CACTACCTTATGTAGTAAAGGTTGAATTTTGAAAGTGGAACTTCAATACCAGATCAATTC

b        *   W   N   T   S   F   P   T   *   N   F   H   L   E   V   M   V   *   L   R   -

             B                   T
             s                   t
             p                   h
             N1                  l
         B   12              BM  l                    M           M
         a   a8              sn  1                    b       B   b
         n   I6              11  I                    o       b   o
         I   VI              II  I                    I       s   I
                                                                  I
        ACATCAGGGGCACCTCCGTTTCTTGGTTTTGTATTGTTTGAAAGAAGACGACATCTTCCT
1681    ---------+---------+---------+---------+---------+---------+  1740
        TGTAGTCCCCGTGGAGGCAAAGAACCAAAACATAACAAACTTTCTTCTGCTGTAGAAGGA

b        H   Q   G   H   L   R   F   L   V   L   Y   C   L   K   E   D   D   I   F   L   -

         B                                               H
         p       C                                       i           C
         uD      AvM                                     n   S   v   P
         1d      lin                                     c   f   i   s
         0e      uJl                                     I   c   R   t
         II      III                                     I   I   I   I
          /       /
        CCTTAGCTGTGTGTTGAAAATGAAAGGGATTGAAAGCACAGTGTCAACTGCAGAATGGTT
1741    ---------+---------+---------+---------+---------+---------+  1800
        GGAATCGACACACAACTTTTACTTTCCCTAACTTTCGTGTCACAGTTGACGTCTTACCAA

b        L   S   C   V   L   K   M   K   G   I   E   S   T   V   S   T   A   E   W   L   -

         H                           R                               C
         iT                          Tl                  M           v
         nf                          ae                  n           i
         fi                          qA                  l           J
         II                          II                  I           I
                                      /
        GATTCTCACTCTGAAAGGATTTACTTCGAGTTATAATGTGGGGGTTAGGAGAGGGGCTGT
1801    ---------+---------+---------+---------+---------+---------+  1860
        CTAAGAGTGAGACTTTCCTAAATGAAGCTCAATATTACACCCCCAATCCTCTCCCCGACA

b        I   L   T   L   K   G   F   T   S   S   Y   N   V   G   V   R   R   G   A   V   -
```

36

FIGURE 11

```
                      N                                    BMT
          T           l                                    sss
          s           a       D       M            H       tep       B
          p           I       d       n            p       EI4       a
          E           I       e       l            h       II5       r
          I           I       I       I            I       III       I
                                                                     /
           TTTTTCCTAATTCCCACCATGTCCTCTAAGTGTTCACAAGGTCAAGTTCAGAAGGTCACC
     1861  --------+--------+---------+--------+---------+--------+  1920
           AAAAAGGATTAAGGGTGGTACAGGAGATTCACAAGTGTTCCAGTTCAAGTCTTCCAGTGG

     b      F   S   *   F   P   P   C   P   L   S   V   H   K   V   K   F   R   R   S   P   -


                          T
          D               a       N
          r               q       l                    C           TM
          a               I       a       D             v       A   sb              E
          i               r       I       d             i       p   po              a
          I               -       I       e             J       o   EI              r
          I               2       I       I             I       o   II              I
                                  I                     I       I                   I
           CAGTGAGTTCATCATGCTATCATTCTGAGCAGGAAGCCAAGAATTTCGCTCTCTTCATTT
     1921  --------+---------+---------+---------+---------+--------+  1980
           GTCACTCAAGTAGTACGATAGTAAGACTCGTCCTTCGGTTCTTAAAGCGAGAGAAGTAAA

     b      S   E   F   I   M   L   S   F   *   A   G   S   Q   E   F   R   S   L   H   F   -


                          T                 C   C                   S
              B           s                 v   a                   f
              s           p                 i   c                   a
              g           E                 R   8                   N
              I           I                 I   I                   I
           TTTTCAGTAATTTCTCCACACTGCACGCTCTTTTGTATTATTTTCCCTGATGCCTTATGA
     1981  --------+---------+---------+---------+---------+--------+  2040
           AAAAGTCATTAAAGAGGTGTGACGTGCGAGAAAACATAATAAAAGGGACTACGGAATACT

     b      F   Q   *   F   L   H   T   A   R   S   F   V   L   F   S   L   M   P   Y   E   -


                                              T
                                              t
                      N  S                     h
                      l  a                     ET1
                      BaDu              B   Acs1
                      cIp3              c   pop1
                      lInA              c   oREI
                      IIII              I   IIII
                         /                  /
           AACAGCATGATCAAACAACAGATGGAATTC
     2041  --------+---------+--------+  2070
           TTGTCGTACTAGTTTGTTGTCTACCTTAAG

     b      T   A   *   S   N   N   R   W   N   -
```

Enzymes that do cut:

| | | | | | | | |
|---|---|---|---|---|---|---|---|
| AciI | AflIII | AluI | AlwI | AlwNI | ApaBI | ApoI | AvaI |
| AvaII | BamHI | BanI | BanII | BbsI | BbvI | BccI | Bce83I |
| BcefI | BclI | BfaI | BpmI | Bpu10I | Bpu1102I | BsaI | BsaAI |
| BsaBI | BsaHI | BsaJI | BsaWI | BsgI | BsiEI | BslI | BsmI |
| BsmAI | Bsp1286I | Bsp1407I | BspGI | BspMI | BspWI | BsrI | BsrFI |
| BstEII | BstXI | BstYI | Cac8I | CviJI | CviRI | DdeI | DpnI |

37

FIGURE 11

```
DraIII     DrdII     DsaI      EarI  Eco47III    Eco57I     EcoRI    EcoRII
 FauI    Fnu4HI     FokI      FspI     HaeII    HaeIII      HgaI    HgiEII
 HhaI    HincII   HindIII    HinfI      HphI     MaeII    MaeIII    MboII
 MlyI      MmeI     MnlI      MseI      MslI      MspI      NciI     NcoI
NlaIII    NlaIV     NspI    NspBII     PflMI      PleI      PstI     PvuI
 PvuII    RleAI     RsaI    Sau96I    Sau3AI      ScaI     ScrFI    SfaNI
 SfcI      SphI     StyI      TaqI    TaqII-1   TaqII-2      TfiI    Tsp45I
 TspEI   Tth111I Tth111II     XcmI      XhoI
```

Enzymes that do not cut:

```
AatII      AccI    AflII      AgeI      ApaI     ApaLI      AscI     AseI
AvrII      BaeI     BcgI      BcgI      BglI     BglII     BscGI     BsiI
BspEI     BspHI    BsrBI    BssHII  Bst1107I    Bsu36I      ClaI     DraI
 DrdI      EaeI     EagI  Eam1105I      EciI     EcoNI  EcoO109I    EcoRV
Esp3I      FinI     FseI     GdiII      HaeI     HgiAI      HpaI     KpnI
 MluI      MscI     MunI      NaeI      NarI      NdeI      NheI     NotI
 NruI      NsiI     NspV      PacI  Pfl1108I      PmeI      PmlI    PshAI
Psp5II  Psp1406I   RsrII      SacI     SacII      SalI      SapI     SfiI
SgrAI      SmaI    SnaBI      SpeI      SrfI  Sse8387I      SspI     StuI
 SunI      SwaI     ThaI      XbaI      XmnI
```

FIGURE 12